(19) Europäisches Patentamt
European Patent Office
Office européen des brevets

(11) **EP 4 304 472 B1**

(12) **FASCICULE DE BREVET EUROPEEN**

(45) Date de publication et mention
de la délivrance du brevet:
**30.10.2024 Bulletin 2024/44**

(21) Numéro de dépôt: **22722318.7**

(22) Date de dépôt: **14.04.2022**

(51) Classification Internationale des Brevets (IPC):
**A61B 5/06** *(2006.01)*    **A61B 5/07** *(2006.01)*
**A61B 5/00** *(2006.01)*    **A61B 5/11** *(2006.01)*
**G06F 3/033** *(2013.01)*    **G06F 3/0346** *(2013.01)*
**G06F 3/01** *(2006.01)*

(52) Classification Coopérative des Brevets (CPC):
**A61B 5/1114; A61B 5/1126; A61B 5/681;
A61B 5/7253; A61B 5/7267; G06F 3/014;
G06F 3/017; G06F 3/0334; G06F 3/0346;**
A61B 5/1122; A61B 2560/0209; A61B 2560/0223;
G06F 2218/08

(86) Numéro de dépôt international:
**PCT/FR2022/050712**

(87) Numéro de publication internationale:
**WO 2022/219289 (20.10.2022 Gazette 2022/42)**

(54) **PROCÉDÉ D'ENCODAGE DE DONNÉES DE CAPTEUR DE MOUVEMENT, DISPOSITIF ET SYSTÈME ASSOCIÉS**

VERFAHREN ZUR CODIERUNG VON BEWEGUNGSSENSORDATEN SOWIE ZUGEHÖRIGE VORRICHTUNG UND SYSTEM

METHOD FOR ENCODING MOTION SENSOR DATA, AND ASSOCIATED DEVICE AND SYSTEM

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **14.04.2021 FR 2103876
14.04.2021 FR 2103875**

(43) Date de publication de la demande:
**17.01.2024 Bulletin 2024/03**

(73) Titulaire: **Zhor Tech
54000 Nancy (FR)**

(72) Inventeurs:
• **CHRETIEN, Mathieu
54000 NANCY (FR)**
• **BERRUET, Brieuc
54000 NANCY (FR)**
• **OUDRHIRI, Radouane
54000 NANCY (FR)**
• **OUMNIA, Karim
54000 NANCY (FR)**

(74) Mandataire: **A.P.I. Conseil
Technopôle Hélioparc
4, rue Jules Ferry
64000 Pau (FR)**

(56) Documents cités:
**US-A1- 2007 061 105    US-A1- 2019 328 243
US-B1- 10 978 093**

## Description

L'invention s'intéresse au domaine de l'étude du déplacement et plus particulièrement au traitement de données brutes provenant de capteurs de mouvement pour faciliter l'étude en temps réel des mouvements, de la position ou plus largement de l'activité d'une entité mobile. En particulier, l'invention s'intéresse au domaine de l'étude de la démarche et plus particulièrement au traitement de données brutes provenant de centrales inertielles pour faciliter l'étude en temps réel des mouvements, de la posture ou plus largement de l'activité d'une personne. L'invention concerne un procédé d'encodage de données générées par un ou plusieurs capteurs de mouvement de préférence couplés à une entité mobile telle qu'une chaussure. L'invention concerne en outre un dispositif d'encodage et un système d'encodage de données générées par un ou plusieurs capteurs de mouvement pouvant mettre en oeuvre un tel procédé.

## [Art antérieur]

L'étude du déplacement trouve des applications dans un grand nombre de domaines différents. En particulier, l'étude du déplacement peut viser à comprendre comment se décomposent les mouvements effectués par une entité lorsqu'elle se déplace. Elle trouve par exemple une application dans le cadre de l'analyse de la démarche humaine, processus complexe et cyclique visant principalement à soutenir la position verticale et à maintenir l'équilibre dans des conditions statiques ou dynamiques. Un tel processus nécessite la synergie des muscles, des os et du système nerveux. En outre, l'étude du mouvement ou du déplacement trouve d'autres applications, par exemple en robotique, afin de permettre la coordination des membres d'un robot, ou bien de manière plus générale pour permettre l'analyse et la représentation des déplacements d'une entité (e.g. objet, individu) au cours du temps.

De nombreux systèmes ont été développés au fil des années pour étudier les mouvements/déplacements d'une entité et par exemple la démarche humaine. Ces systèmes comportent par exemple l'utilisation de capteurs de pression ou de capteurs inertiels positionnés comme par exemple dans la semelle d'une chaussure, sur la chaussure ou encore au niveau de la cheville. En particulier, plusieurs équipes se sont concentrées sur l'utilisation et le traitement des données de capteurs inertiels pour l'analyse du mouvement humain ou plus largement l'analyse d'entité mobile telle que des drones. Ces systèmes, basés sur un ou plusieurs capteurs, fournissent aujourd'hui des valeurs de paramètres « marche » classiques indiquant par exemple des informations sur le nombre de pas grâce à des calculs réalisés le plus souvent intégralement sur des terminaux externes.

En outre, ces systèmes reposent le plus souvent sur des capteurs de pression ou des capteurs inertiels couplés à des centrales de calcul déportées dans des dispositifs externes présentant de fortes capacités de calcul. Ainsi, les systèmes d'étude du mouvement ou de la démarche actuels comportent généralement la transmission de données à haute fréquence depuis les capteurs embarqués vers les centrales de calcul. Ces systèmes doivent être capables de fonctionner en continu sans avoir à changer/recharger la batterie fréquemment tout en proposant une analyse fine du mouvement ou de la démarche d'un utilisateur. Or, dans les systèmes classiques, la transmission en temps réel des données brutes entraine une forte consommation énergétique alors que ces systèmes doivent pouvoir fonctionner en autonomie sur une longue durée. Par exemple, dans le cadre d'une solution pour suivre l'activité d'un utilisateur en analysant les données des capteurs de mouvements, il a été proposé de coder les données de tension générées par les centrales inertielles sur 8 bits (US2007061105). Toutefois, une telle solution vient réduire la précision des données de tension transmises pouvant avoir un impact sur la précision globale de la solution. Alternativement, la transmission de données prétraitées exclusivement limite les possibilités d'évolution des interprétations des données mesurées par les capteurs de mouvement. En outre, il a été proposé une solution de reconnaissance de mouvement combinant l'analyse de données d'un capteur de mouvement avec des données audio (US10978093).

Une étude réalisée en 2018 montre que sur les 14 systèmes évalués, la médiane d'autonomie des systèmes proposés est située à moins de 6 heures. Cela est clairement insuffisant pour des applications grand public où un suivi de la démarche d'un individu sur une journée au moins est souhaité (« A survey on smart shoe insole systems ». Salma Saidani et al. 2018 10.1109/SMARTNETS.2018.8707391 ; ©2018 IEEE).

Une solution pour augmenter l'autonomie des systèmes d'étude de la démarche d'un individu est d'augmenter la taille de la batterie. Néanmoins, une telle solution ne peut pas être appliquée dans toutes les situations, notamment lorsqu'une miniaturisation des dispositifs de suivi est souhaitée. Il a par exemple été proposé des centrales inertielles capables de fonctionner pendant environ 35 heures en envoyant les données brutes à un dispositif externe capable de les analyser (« Bluetooth Embedded Inertial Measurement Unit for Real-Time Data Collection for Gait Analysis ». Nimsiri Abhayasinghe et al. Octobre 2013 ; Conference: Indoor Positioning and Indoor Navigation. Montbéliard (France)). Toutefois une telle centrale inertielle présente des dimensions de l'ordre de 50 cm$^3$ et un poids de l'ordre de 50 grammes. De telles dimensions sont notamment imposées par l'utilisation d'une batterie présentant une capacité suffisante pour transmettre en continu les données à un dispositif externe de traitement.

D'autres solutions, déjà proposées par la demanderesse (WO2019193301, WO2019077266, WO2020217037), sont de permettre un calcul au moins partiel au niveau du dispositif de mesure du mouvement de façon à ne pas transférer l'ensemble des données mesurées et ainsi réduire la consommation associée au stockage et/ou transfert de données. Néanmoins, une telle solution présume de disposer de tous les algorithmes déjà intégrés dans le dispositif de mesure. Or, dans certains cas, seulement une partie des algorithmes de traitement de données ont été intégrés dans le dispositif de mesure. Il peut aussi exister un besoin de faire évoluer les méthodes de traitement des données.

Ainsi, il existe un besoin pour une méthode d'encodage des données de capteur de mouvement permettant un stockage et un transfert de données qui soit moins énergivore au niveau du dispositif de mesure tout en minimisant au maximum la perte de données.

**[Problème technique]**

L'invention a pour but de remédier aux inconvénients de l'art antérieur. En particulier, l'invention a pour but de proposer un procédé d'encodage de données générées par un ou plusieurs capteurs de mouvement de préférence couplé(s) à une entité mobile ou une chaussure, capable de générer/transmettre une information fidèle des données mesurées et suffisante pour l'étude du mouvement de l'entité mobile équipée par lesdits capteurs de mouvements ou de la démarche de l'individu portant lesdits capteurs de mouvements. Un tel procédé permet à des systèmes de fonctionner sur de plus longues durées sans avoir à changer/recharger la batterie, cela tout en proposant une analyse fine du mouvement et en particulier de la démarche d'un utilisateur. En outre, un tel procédé rend possible une détermination du mouvement d'une entité mobile ou de la démarche d'un utilisateur en temps réel.

L'invention a en outre pour but de proposer un dispositif d'encodage de données et un système de détermination du mouvement d'une entité mobile ou de la démarche d'un utilisateur intégrant un tel dispositif, ledit système présentant une autonomie améliorée tout en étant capable d'une étude du mouvement de l'entité mobile ou de la démarche de l'utilisateur en temps réel.

**[Brève description de l'invention]**

L'invention vise à pallier ces inconvénients. Ce qui suit présente un résumé simplifié d'aspects, de modes de réalisation et d'exemples sélectionnés de la présente invention dans le but de fournir une compréhension de base de l'invention. Cependant, ce résumé ne constitue pas un aperçu exhaustif de tous les aspects, modes de réalisation et exemples de l'invention. Son seul but est de présenter des aspects, modes de réalisation et exemples sélectionnés de l'invention sous une forme concise en guise d'introduction à la description plus détaillée des aspects, modes de réalisation et exemples de l'invention qui suivent le résumé.

À cet effet, l'invention porte sur un procédé d'encodage de données générées par un ou plusieurs capteurs de mouvement couplé(s) à une entité mobile, pour l'identification d'une position ou d'un mouvement de l'entité mobile sur une séquence temporelle, ledit procédé, exécuté par un ou plusieurs processeurs, comportant :

- Une étape d'acquisition de données générées par le ou les capteurs de mouvement couplé(s) à l'entité mobile, lesdites données comportant des valeurs variables sous forme de séries temporelles, de préférence lesdites valeurs variables comportent des valeurs d'accélération et de vitesse angulaire ;
- Une étape de calcul, à partir des données acquises sur une fenêtre temporelle, d'une pluralité de descripteurs, ladite étape de calcul étant répétée pour plusieurs fenêtres temporelles consécutives, les fenêtres temporelles consécutives formant la séquence temporelle ; et
- Une étape de transformation de la pluralité de descripteurs de chacune des fenêtres temporelles consécutives formant la séquence temporelle, ladite étape de transformation comportant une génération pour chacun des descripteurs d'une donnée codée sur au plus huit bits, de préférence sur au plus quatre bits ;

lesdites données codées sur au plus huit bits associées à la séquence temporelle étant représentatives d'une position ou d'un mouvement de l'entité mobile sur ladite séquence temporelle.

En particulier, l'invention porte sur un procédé d'encodage de données générées par un ou plusieurs capteurs de mouvement couplés à une chaussure, pour l'identification d'une position ou d'un mouvement de la chaussure sur une séquence temporelle, ledit procédé, exécuté par un ou plusieurs processeurs, comportant :

- Une étape d'acquisition de données générées par le ou les capteurs de mouvement couplé à la chaussure, lesdites données comportant des valeurs variables sous forme de séries temporelles, de préférence lesdites valeurs variables comportent des valeurs d'accélération et de vitesse angulaire ;
- Une étape de calcul, à partir des données acquises sur une fenêtre temporelle, d'une pluralité de descripteurs, ladite étape de calcul étant répétée pour plusieurs fenêtres temporelles consécutives, les fenêtres temporelles consécutives formant la séquence temporelle ; et
- Une étape de transformation de la pluralité de descripteurs de chacune des fenêtres temporelles consécutives formant la séquence temporelle, ladite étape de transformation comportant une génération, pour chacun des descripteurs, d'une donnée codée sur au plus huit bits, de préférence sur au plus quatre bits ;

lesdites données codées sur au plus huit bits associées à la séquence temporelle étant représentatives d'une position ou d'un mouvement de la chaussure sur ladite séquence temporelle.

La demanderesse a développé un procédé embarquable capable de traiter quasi instantanément (e.g. temps inférieur à 1 ms avec une cadence de processeur à 64 MHz), de préférence par des processeurs couplés aux capteurs de mouvements, les données brutes générées par les capteurs de mouvement. Les capteurs de mouvement peuvent être positionnés au niveau d'une chaussure (sur la chaussure, dans une semelle...) qui est portée par un utilisateur. Cela permet de les encoder dans un format qui pourra être soit traité au niveau d'un processeur couplé au capteur de mouvement, par exemple dans un dispositif embarqué tel qu'un bracelet ou un téléphone ou une semelle, soit transmis ponctuellement à un terminal externe avec une consommation d'énergie réduite et/ou une mémoire de stockage réduite.

La demanderesse a développé en particulier un encodage de données permettant une meilleure interprétation par apprentissage automatique des mouvements. Sans être lié par la théorie, cet encodage peut être considéré comme une phonémisation des séries temporelles issues des capteurs de mouvement. Ainsi, le procédé générera à partir d'une série temporelle de données de capteur de mouvement, un ensemble de « phonème » capable de décrire des intervalles consécutifs de la série temporelle. La combinaison ou l'assemblage particulier de ces phonèmes permettra de définir des positions, des postures ou des mouvements particuliers.

En outre, un tel encodage donne également la possibilité à un algorithme de traiter la donnée encodée en temps réel pour la traduire en des prédictions de déplacements d'une entité mobile ou de démarche d'un individu (e.g. mouvement et/ou posture).

Les données ainsi encodées pourront être alors traitées ou transmises sous la forme de tenseur de données. Le procédé comportant alors une étape de génération d'un tenseur de données comportant les données codées sur au plus huit bits obtenus pour plusieurs des fenêtres temporelles, de sorte que le tenseur de données comporte un encodage représentatif d'une position, d'une posture ou d'un mouvement de l'entité mobile ou de la chaussure. En outre, le tenseur de données pourra comporter un encodage représentatif d'une pluralité de positions, postures ou de mouvement de l'entité mobile ou de la chaussure.

**Selon d'autres caractéristiques optionnelles du procédé, ce dernier peut inclure facultativement une ou plusieurs des caractéristiques suivantes, seules ou en** combinaison :

- les un ou plusieurs capteurs de mouvement ainsi que les un ou plusieurs processeurs sont intégrés dans un dispositif d'encodage, de préférence positionné dans un accessoire porté par un utilisateur telle qu'une montre ou un bracelet. Lorsque l'entité mobile est un objet, le dispositif d'encodage pourra par exemple être intégré aux circuits électroniques contrôlant le déplacement de l'objet ou bien dans un dispositif électronique dédié. En particulier, les un ou plusieurs capteurs de mouvement ainsi que les un ou plusieurs processeurs sont intégrés dans un dispositif d'encodage, de préférence positionné dans une semelle. Un tel dispositif embarquant capteur de mouvement et processeurs permet de réaliser une partie du traitement au plus proche de la génération d'une donnée brute de mouvement et donc d'accélérer le traitement et d'en réduire le cout énergétique.

- Le dispositif d'encodage est positionné dans une montre ou un bracelet. Alternativement, le dispositif d'encodage est positionné dans une semelle amovible. La présence du dispositif d'encodage dans une montre ou un bracelet ou une semelle amovible est possible grâce à la faible consommation du procédé et permet d'utiliser le procédé sur plusieurs accessoires tels que des articles chaussants.

- l'étape de calcul comporte un prétraitement des données acquises. Il peut être avantageux de modifier les données, par exemple, via des filtrages, de façon à accélérer le traitement ultérieur et dans certains cas réduire la consommation d'énergie associée.

- il comporte en outre une étape de génération d'un tenseur de données comportant les données codées sur au plus huit bits obtenus pour plusieurs des fenêtres temporelles, de sorte que le tenseur de données comporte un encodage représentatif d'une position ou d'un mouvement de l'entité mobile.

- les descripteurs sont calculés à partir de fonctions mathématiques appliquées aux données, prétraitées ou non, générées par le ou les capteurs de mouvement couplé à l'entité mobile telle que la chaussure, et lesdites fonctions mathématiques appliquées comportant par exemple un calcul de moyenne, d'asymétrie statistique, du coefficient d'aplatissement, de comparaisons de valeurs des extremums ou des quartiles, de positions des extremums dans une série temporelle, de comparaisons des valeurs de bord des sous-ensembles du mouvement et/ou d'un calcul d'une topologie de données.

- il comporte une étape d'extraction d'une séquence temporelle pouvant correspondre à une unité de mouvement, ladite étape d'extraction mettant en oeuvre un modèle d'apprentissage, de préférence un modèle d'apprentissage non supervisé. Une extraction préalable d'une unité de mouvement peut permettre d'améliorer la justesse de prédiction du procédé tout en réduisant la consommation d'énergie associée.

- Il comporte une étape d'analyse des données codées sur au plus huit bits, de préférence du tenseur de données, en fonction d'une donnée d'activité de l'entité mobile, ladite étape d'analyse comprenant une comparaison entre les données codées sur au plus huit bits ou de préférence le tenseur de données et une base de données comprenant des données de référence pour l'activité donnée. En particulier, il comporte une étape d'analyse des données codées sur au plus huit bits, de préférence du tenseur de données, en fonction d'une donnée d'activité de l'utilisateur, ladite étape d'analyse comprenant une comparaison entre les données codées sur au plus huit bits ou de préférence le tenseur de données et une base de données comprenant des données de référence pour l'activité donnée. Cela permet avantageusement de déterminer quelles données sont associées aux mouvements relatifs à une activité et quelles données correspondent à des mouvements à risque ou non intentionnels. L'activité donnée peut par exemple correspondre à un protocole de déplacement, un protocole de fonctionnement, une activité sportive mais également une activité de détente ou professionnelle.
- les fenêtres temporelles de deux séquences temporelles consécutives présentent des durées différentes. Ainsi, l'analyse peut s'adapter à différents types de déplacements, différentes temporalités de cycle de déplacements et ne nécessite pas de longue segmentation des données. En particulier, l'analyse peut s'adapter à différents types de marches, différentes temporalité de cycle de marche et ne nécessite pas de longue segmentation des données.
- lors de l'étape de transformation, au moins quatre descripteurs sont utilisés pour chacun générer une donnée codée sur au moins un bit, de façon plus préférée sur au moins deux bits.
- lors de l'étape de transformation, plusieurs descripteurs sont transformés en une seule donnée d'au plus huit bits, de préférence d'au plus trois bits. Dans certains cas, des descripteurs « composites », formés à partir de plusieurs descripteurs peuvent être les seuls à être nécessaires. En particulier, lors de l'étape de transformation, au moins huit descripteurs sont utilisés, pour chacun générer une donnée codée sur au moins un bit, de façon plus préférée au moins deux bits.
- une ou plusieurs données codées sur au plus huit bits, de préférence au plus trois bits, sont calculées à partir de valeurs de descripteurs calculés à partir de données acquises sur des fenêtres temporelles consécutives. Dans certains cas, un descripteur « composite », formé à partir de plusieurs descripteurs provenant de fenêtres temporelles différentes peut améliorer la performance.
- lors de l'étape de transformation, entre quatre et vingt descripteurs sont utilisés pour chacun générer une donnée codée sur un nombre de bits sélectionné parmi : un, deux ou trois. De telles valeurs permettent d'obtenir de meilleures performances en termes de conservation de données, volume de stockage et consommation électrique.
- l'entité mobile est une chaussure, et l'étape d'acquisition est réalisée alors que les un ou plusieurs capteurs de mouvement ainsi que les un ou plusieurs processeurs sont intégrés dans un dispositif d'encodage, de préférence positionné dans une semelle.

Selon **un deuxième objet,** l'invention porte sur un procédé de détermination d'un déplacement d'une entité mobile, à partir de données susceptibles d'avoir été encodées selon un procédé d'encodage selon l'invention, ledit procédé de détermination, exécuté par un ou plusieurs processeurs, comportant :

- Une étape de chargement d'un ou de plusieurs tenseurs de données comportant un encodage représentatif d'une position ou d'un mouvement de l'entité mobile, de préférence lesdits un ou de plusieurs tenseurs de données comportant une pluralité de descripteurs pour chacune des fenêtres temporelles consécutives formant une séquence temporelle, chacun des descripteurs correspondant à une donnée codée sur au plus huit bits ; et
- Une étape de traitement du ou des tenseurs de données par un modèle d'apprentissage automatique entrainé de manière à identifier une position ou un mouvement de l'entité mobile.

En particulier, l'invention porte sur un procédé de détermination de la démarche d'un utilisateur d'une chaussure, à partir de données susceptibles d'avoir été encodées selon un procédé d'encodage selon l'invention, ledit procédé de détermination, exécuté par un ou plusieurs processeurs, comportant :

- Une étape de chargement d'un ou de plusieurs tenseurs de données comportant un encodage représentatif d'une position ou d'un mouvement de la chaussure de l'utilisateur, de préférence lesdits un ou de plusieurs tenseurs de données comportant une pluralité de descripteurs pour chacune des fenêtres temporelles consécutives formant une séquence temporelle, chacun des descripteurs correspondant à une donnée codée sur au plus huit bits ; et
- Une étape de traitement du ou des tenseurs de données par un modèle d'apprentissage automatique entrainé de manière à identifier une posture ou un mouvement de l'utilisateur.

**Selon d'autres caractéristiques optionnelles du procédé, ce dernier peut inclure facultativement une ou plusieurs des caractéristiques suivantes, seules ou en combinaison** :

- le déplacement de l'entité mobile comporte un ou plusieurs mouvements et/ou positions de l'entité mobile. En particulier, le déplacement de l'entité mobile peut correspondre à la démarche de l'utilisateur. La démarche de l'utilisateur comporte de préférence un ou plusieurs mouvements et/ou postures de l'utilisateur.
- l'étape de traitement du ou des tenseurs de données par un modèle d'apprentissage automatique comporte l'identification de classes d'événements à partir d'un ou de plusieurs tenseurs de données, de préférence une classe de mouvement par séquence temporelle.
- il comporte une étape de sélection, de préférence par le modèle d'apprentissage automatique entrainé, d'une position ou d'un mouvement à partir d'une comparaison des positions ou mouvements identifiés à partir de fenêtres temporelles contiguës ou de séquences temporelles contiguës. En particulier, il comporte une étape de sélection, de préférence par le modèle d'apprentissage automatique entrainé, d'une posture ou d'un mouvement de l'utilisateur à partir d'une comparaison des postures ou mouvements de l'utilisateur identifiés à partir de fenêtres temporelles contiguës ou de séquences temporelles contiguës.
- il comporte l'identification, par le modèle d'apprentissage entrainé, d'une position ou d'un mouvement pour chacune de plusieurs séquences temporelles contiguës, le procédé comportant alors une sélection de la position ou du mouvement le plus représenté au sein des séquences temporelles contiguës. En particulier, il comporte l'identification, par le modèle d'apprentissage entrainé, d'une posture ou d'un mouvement pour chacune de plusieurs séquences temporelles contiguës, le procédé comportant alors une sélection de la posture ou du mouvement le plus représenté au sein des séquences temporelles contiguës. Une telle étape permet de réduire les risques de faux positifs en tirant parti de la reconnaissance en temps réel de séries temporelles et d'une validation multiple d'une même position ou posture ou mouvement. Par exemple, un mouvement peut être sélectionné à partir d'une analyse d'au moins 3, de préférence d'au moins 4 et de façon encore plus préférée d'au moins cinq séquences temporelles contiguës.
- il comporte une étape de comparaison de positions et/ou de mouvements identifiés pour une fenêtre temporelle ou une séquence temporelle donnée à partir de données de capteurs différents de façon à identifier une position ou un mouvement de l'entité mobile. En particulier, il comporte une étape de comparaison de postures et/ou de mouvements de l'utilisateur identifiés pour une fenêtre temporelle ou une séquence temporelle donnée à partir de données de capteurs différents de façon à identifier une posture ou d'un mouvement de l'utilisateur. Par exemple, il peut comporter, pour une unité de mouvement, une comparaison du mouvement identifié pour chacun des bras, des pieds, ou une comparaison par rapport à un référentiel, de façon à déterminer une position, une posture ou un mouvement comme ceux d'un utilisateur couplé aux capteurs de mouvements.
- l'entité mobile est un utilisateur d'une chaussure, l'encodage représentatif d'une position ou d'un mouvement de l'entité mobile est un encodage représentatif d'une position ou d'un mouvement de la chaussure de l'utilisateur ; et l'étape de traitement du ou des tenseurs de données permet d'identifier une posture ou un mouvement de l'utilisateur.

L'invention porte en outre sur un dispositif d'encodage de données générées par un ou plusieurs capteurs de mouvement couplés à une entité mobile, pour l'identification d'une position ou d'un mouvement de l'entité mobile sur une séquence temporelle, ledit dispositif d'encodage comportant un ou plusieurs processeurs, lesdits un ou plusieurs processeurs étant configurés pour :

- Acquérir les données générées par un ou plusieurs capteurs de mouvement couplé(s) à l'entité mobile , lesdites données comportant des valeurs variables sous forme de séries temporelles, de préférence des valeurs d'accélération et de vitesse angulaire ;
- Calculer, à partir des données acquises sur une fenêtre temporelle, une pluralité de descripteurs, le calcul étant répété pour plusieurs fenêtres temporelles consécutives, les fenêtres temporelles consécutives formant une séquence temporelle ;
- Transformer la pluralité de descripteurs de chacune des fenêtres temporelles consécutives formant la séquence temporelle, ladite transformation comportant une génération pour chacun des descripteurs d'une donnée codée sur au plus huit bits, de préférence au plus trois bits, lesdites données codées sur au plus huit bits associées à une fenêtre temporelle étant représentatives d'un mouvement ou d'une position de l'entité mobile sur ladite séquence temporelle.

En particulier, l'invention porte en outre sur un dispositif d'encodage de données générées par un ou plusieurs capteurs de mouvement couplé(s) à une chaussure, pour l'identification d'une position ou d'un mouvement de la chaussure sur une séquence temporelle, ledit dispositif d'encodage comportant un ou plusieurs processeurs, lesdits un ou plusieurs processeurs étant configurés pour :

- Acquérir les données générées par le ou les capteurs de mouvement couplé(s) à la chaussure, lesdites données comportant des valeurs variables sous forme de séries temporelles, de préférence des valeurs d'accélération et de vitesse angulaire ;

- Calculer, à partir des données acquises sur une fenêtre temporelle, une pluralité de descripteurs, le calcul étant répété pour plusieurs fenêtres temporelles consécutives, les fenêtres temporelles consécutives formant une séquence temporelle ;
- Transformer la pluralité de descripteurs de chacune des fenêtres temporelles consécutives formant la séquence temporelle, ladite transformation comportant une génération pour chacun des descripteurs d'une donnée codée sur au plus huit bits, de préférence d'au plus trois bits, lesdites données codées sur au plus huit bits associées à une fenêtre temporelle étant représentatives d'un mouvement de la chaussure sur ladite séquence temporelle.

En outre, le dispositif d'encodage peut comporter lesdits un ou plusieurs capteurs de mouvement. De préférence, les un ou plusieurs capteurs de mouvement comportent au moins un accéléromètre et au moins un gyroscope. En outre, le ou les processeurs peuvent être configurés pour générer un tenseur de données comportant les données codées sur au plus huit bits obtenus pour plusieurs des fenêtres temporelles, de sorte que le tenseur de données comporte un encodage représentatif d'une position ou d'un mouvement de l'entité mobile. Par exemple, le ou les processeurs peuvent être configurés pour générer un tenseur de données comportant les données codées sur au plus huit bits obtenus pour plusieurs des fenêtres temporelles, de sorte que le tenseur de données comporte un encodage représentatif d'une position ou d'un mouvement de la chaussure. Le dispositif d'encodage peut être intégré dans une semelle amovible ou non.

L'invention porte en outre sur un système de détermination d'une position ou d'un mouvement d'une entité mobile, ledit système de détermination comportant au moins un dispositif d'encodage selon l'invention et un terminal externe configuré pour recevoir le tenseur de données généré puis pour déterminer le déplacement d'une entité mobile à partir du tenseur de données généré. En particulier, l'invention porte en outre sur un système de détermination de la démarche d'un utilisateur, ledit système de détermination comportant au moins un dispositif d'encodage selon l'invention et un terminal externe configuré pour recevoir le tenseur de données généré puis pour déterminer la démarche d'un utilisateur à partir du tenseur de données généré.

D'autres avantages et caractéristiques de l'invention apparaitront à la lecture de la description suivante donnée à titre d'exemple illustratif et non limitatif, en référence aux Figures annexées :

La figure 1 représente un schéma d'un procédé selon un mode de réalisation de l'invention. Les étapes en pointillés sont facultatives.

La figure 2 représente un schéma d'un procédé selon un autre mode de réalisation de l'invention. Les étapes en pointillés sont facultatives.

Les figures 3A et 3B représentent chacune un graphe montrant des données générées, divisées en séquence temporelle subdivisée en fenêtres temporelles, par un capteur de mouvement selon l'axe x. La figure 3C correspond à des tenseurs de données d'ordre 2 et des tenseurs issus respectivement de données de fenêtres temporelles et de données de séquences temporelles.

La figure 4 représente un schéma d'un procédé de détermination du déplacement d'une entité mobile ou de la démarche d'un utilisateur selon un mode de réalisation de l'invention. Les étapes en pointillés sont facultatives.

La figure 5 représente un schéma d'un dispositif d'encodage ouvert vu du dessus comprenant notamment une carte électronique, une source d'énergie, un connecteur et un moyen de communication.

Les figure 6A et 6B représente un schéma d'un système de détermination d'un déplacement d'une entité mobile selon l'invention. En particulier, la figure 6B représente un schéma d'un système de détermination de la démarche d'un utilisateur selon l'invention.

Des aspects de la présente invention sont décrits en référence à des organigrammes et/ou à des schémas fonctionnels de procédés, d'appareils (systèmes) et de produits de programme d'ordinateur selon des modes de réalisation de l'invention.

Sur les figures, les organigrammes et les schémas fonctionnels illustrent l'architecture, la fonctionnalité et le fonctionnement d'implémentations possibles de systèmes, de procédés et de produits de programme d'ordinateur selon divers modes de réalisation de la présente invention. À cet égard, chaque bloc dans les organigrammes ou blocs-diagrammes peut représenter un système, un dispositif, un module ou un code, qui comprend une ou plusieurs instructions exécutables pour mettre en oeuvre la ou les fonctions logiques spécifiées. Dans certaines implémentations, les fonctions associées aux blocs peuvent apparaitre dans un ordre différent que celui indiqué sur les figures. Par exemple, deux blocs montrés successivement peuvent, en fait, être exécutés sensiblement simultanément, ou les blocs peuvent parfois être exécutés dans l'ordre inverse, en fonction de la fonctionnalité impliquée. Chaque bloc des schémas de principe et/ou de l'organigramme, et des combinaisons de blocs dans les schémas de principe et/ou l'organigramme, peuvent être mis en oeuvre par des systèmes matériels spéciaux qui exécutent les fonctions ou actes spécifiés ou effectuer des combinaisons de matériel spécial et d'instructions informatiques.

**[Description de l'invention]**

Dans la suite de la description, le « **déplacement** » au sens de l'invention correspond à la position, les mouvements, la locomotion, et l'équilibre de l'entité mobile. L'équilibre correspond notamment à la stabilité du par rapport au centre de gravité de l'entité mobile. Néanmoins il peut intégrer aussi bien l'équilibre statique que l'équilibre dynamique. La « **démarche** » au sens de l'invention correspond de préférence à la posture, les mouvements, la locomotion, et l'équilibre de l'utilisateur. L'équilibre correspond notamment à l'équilibre postural lié à la stabilité du corps et plus particulièrement à la stabilité du centre de gravité d'un utilisateur. Néanmoins il peut intégrer aussi bien l'équilibre statique que l'équilibre dynamique.

La « **détermination d'un déplacement** » correspond, au sens de l'invention, à l'attribution d'une ou de plusieurs valeurs par exemple un score, un classement ou une note à une trajectoire ou au mouvement de l'entité mobile, et comme présenté en lien avec un mode de réalisation, d'un bras d'un utilisateur. Cette détermination du déplacement peut aussi permettre d'obtenir une ou plusieurs valeurs numériques ou alphanumériques de paramètres biomécanique représentatifs du déplacement en fonction de l'entité mobile considérée. La « **détermination de la démarche** » correspond, au sens de l'invention, à l'attribution d'une ou de plusieurs valeurs par exemple un score, un classement ou une note à une trajectoire ou au déplacement d'un pied d'un utilisateur. Cette détermination de la démarche peut aussi permettre d'obtenir une ou plusieurs valeurs numériques ou alphanumériques de paramètres biomécanique représentatifs de la démarche. Une solution (e.g. procédé, dispositif, système...) selon l'invention pourra permettre de déterminer une position, une posture ou un mouvement de l'entité mobile. Dans la présente demande et pour l'ensemble de la description, de préférence, l'invention permet de déterminer un mouvement d'une entité mobile en effet une succession de position permet d'en déduire un mouvement. Dans le cadre de l'étude d'une démarche d'un utilisateur, l'invention pourra être utilisée pour déterminer une posture ou une démarche d'un utilisateur et de préférence une démarche d'un utilisateur.

Le terme « **activité** » au sens de l'invention peut correspondre à une action réalisée par l'entité mobile ou l'utilisateur sur une période prolongée. Cela peut par exemple correspondre à un type de déplacement particulier, lié à titre d'exemples non limitatifs à une activité sportive telle que du football, du cyclisme ou du basketball, mais aussi à une activité sédentaire telle qu'une activité de bureau ou toute autre activité telle qu'une marche récréative ou une randonnée pédestre.

On entend par « **entité mobile** », toute entité capable de, ou configurée pour, se déplacer de manière autonome ou non. Une entité mobile peut par exemple correspondre à un être vivant, un véhicule, un engin robotisé, une chaussure ou encore un drone.

Le terme « **Descripteur** » au sens de l'invention peut correspondre de préférence à une valeur obtenue via une transformation mathématique des données acquises à partir des capteurs de mouvement. Une transformation mathématique pourra par exemple correspondre au calcul d'une moyenne, d'une médiane, d'une variance, d'asymétrie statistique, du coefficient d'aplatissement, de valeurs des extremums, de valeurs de quartile, de comparaisons de valeurs des extremums ou des quartiles, de positions des extremums dans une série temporelle, et de comparaisons des valeurs de bord des sous-ensembles du mouvement. Par exemple, pour une fenêtre, une valeur de bord correspond à la valeur initiale et finale de la fenêtre.

L'expression « **phonème de mouvement** » au sens de l'invention, peut correspondre à un ensemble de données calculées à partir de plusieurs descripteurs générés sur une fenêtre temporelle donnée. Ces données prennent avantageusement la forme dans le cadre de l'invention de données encodées sur au plus huit bits. Ainsi le phonème de mouvement correspond à plusieurs valeurs encodées sur au plus huit bits décrivant au moins en partie un mouvement sur la fenêtre temporelle étudiée. Ces phonèmes de mouvement peuvent ainsi correspondre à des données calculées à partir de plusieurs descripteurs décrivant une sous-partie d'un mouvement appelée un micromouvement, c'est-à-dire un mouvement pouvant par exemple se dérouler sur moins de 500 ms.

L'expression « **tenseur de données** » au sens de l'invention, peut correspondre à un objet, ou à une structure de données, telle qu'un tableau à une dimension, une matrice à deux dimensions ou encore un cube comportant des données relatives à des valeurs de descripteurs transformées en valeurs codées en au plus huit bits, de préférence en au plus deux bits. La représentation sous forme de tenseur est une représentation des données permettant par exemple d'avoir recours à des opérations de calcul matriciel, optimisée en termes de complexité de calcul et mémoire. En outre, les tenseurs de données, lorsqu'ils correspondent aux valeurs d'un capteur pour une fenêtre temporelle peuvent former un tenseur de données d'ordre 3 et peuvent être traités conjointement avec des tenseurs de données pour d'autres capteurs et des tenseurs de données pour d'autres fenêtres temporelles de façon à former une liste de tenseurs de données d'ordre 3.

Par « **paramètre bio-mécanique** », on entend au sens de l'invention une caractéristique de la démarche d'une entité mobile lorsque celle-ci correspond à un être humain ou à un robot dont les déplacements nécessitent une coordination particulière, par exemple la démarche de l'utilisateur.

Le terme « **accessoire** », correspond à tout article destiné à être porté par une entité mobile. Un accessoire selon l'invention peut prendre la forme d'un article textile ou plus généralement d'un vêtement. L'accessoire peut également être adapté pour être fixé ou intégré à un vêtement ou prendre la forme d'un bracelet. L'accessoire peut également être un élément modulaire amovible adaptable sur un ski, des bâtons de marche, une chaussure (par exemple une cale pour le cyclisme), une prothèse etc.

On entend par « **semelle** » un objet permettant de séparer le pied de l'utilisateur du sol. Une chaussure peut comporter une couche de semelle supérieure en contact direct avec le pied de l'utilisateur et une couche de semelle inférieure en contact direct avec le sol ou plus généralement l'environnement extérieur. Une chaussure peut aussi comporter une semelle interne amovible.

On entend par « **amovible** » la capacité à être détachée, enlevée ou démontée aisément sans avoir à détruire des moyens de fixation soit parce qu'il n'y a pas de moyens de fixation soit parce que les moyens de fixation sont aisément et rapidement démontables (e.g. encoche, vis, languette, ergot, clips). Par exemple, par amovible, il faut comprendre que l'objet n'est pas fixé par soudure ou par un autre moyen non prévu pour permettre de détacher l'objet.

Par « **modèle** » ou « **règle** » ou « **algorithme** », il faut comprendre au sens de l'invention une suite finie d'opérations ou d'instructions permettant de calculer une valeur par l'intermédiaire d'un classement ou d'un partitionnement des données au sein de groupes préalablement définis Y et d'attribuer un score ou de hiérarchiser une ou plusieurs données au sein d'un classement. La mise en oeuvre de cette suite finie d'opérations permet par exemple d'attribuer une étiquette Y à une observation décrite par un ensemble de caractéristiques ou paramètres X grâce par exemple à la mise en oeuvre d'une fonction f, susceptible de reproduire Y en ayant observé X.

$$Y = f(X) + e$$

où e symbolise le bruit ou erreur de mesure.

Par « **méthode d'apprentissage supervisé** », on entend au sens de l'invention un procédé permettant de définir une fonction f à partir d'une base de n observations étiquetées $(X_{1...n}, Y_{1...n})$ où $Y = f(X) + e$.

Par « **méthode d'apprentissage non supervisée** », on entend une méthode visant à hiérarchiser les données ou à diviser un ensemble de données en différents groupes homogènes, les groupes de données homogènes partageant des caractéristiques communes et cela sans que les observations soient étiquetées.

On entend par « **sensiblement constante** » une valeur variant de moins de 15 % par rapport à la valeur comparée, de préférence de moins de 10 %, de façon encore plus préférée de moins de 5 %.

On entend par « **traiter** », « **calculer** », « **déterminer** », « **afficher** », « **transformer** », « **extraire** », « **comparer** » ou plus largement « **opération exécutable** », au sens de l'invention, une action effectuée par un dispositif ou un processeur sauf si le contexte indique autrement. À cet égard, les opérations se rapportent à des actions et/ou des processus d'un système de traitement de données, par exemple un système informatique ou un dispositif informatique électronique, qui manipule et transforme les données représentées en tant que quantités physiques (électroniques) dans les mémoires du système informatique ou d'autres dispositifs de stockage, de transmission ou d'affichage de l'information. Ces opérations peuvent se baser sur des applications ou des logiciels.

Les termes ou expressions « **application** », « **logiciel** », « **code de programme** », et « **code exécutable** » signifient toute expression, code ou notation, d'un ensemble d'instructions destinées à provoquer un traitement de données pour effectuer une fonction particulière directement ou indirectement (e.g. après une opération de conversion vers un autre code). Les exemples de code de programme peuvent inclure, sans s'y limiter, un sous-programme, une fonction, une application exécutable, un code source, un code objet, une bibliothèque et/ou toute autre séquence d'instructions conçues pour l'exécution sur un système informatique.

On entend par « **processeur** », au sens de l'invention, au moins un circuit matériel configuré pour exécuter des opérations selon des instructions contenues dans un code. Le circuit matériel peut être un circuit intégré. Des exemples d'un processeur comprennent, sans s'y limiter, une unité de traitement central, un processeur graphique, un circuit intégré spécifique à l'application (ASIC) et un circuit logique programmable.

On entend par « **dispositif informatique** », tout dispositif comprenant une unité de traitement ou un processeur, par exemple sous la forme d'un microcontrôleur coopérant avec une mémoire de données, éventuellement une mémoire programme, lesdites mémoires pouvant être dissociées. L'unité de traitement coopère avec lesdites mémoires au moyen d'un bus de communication interne.

Par « **infrastructure informatique** », on entend au sens de l'invention un ensemble de structures informatiques (i.e. dispositifs informatiques) apte à faire fonctionner une application ou une chaine applicative. La structure informatique peut être un serveur et peut par exemple être composée d'un serveur de présentation, d'un serveur métier et d'un serveur de données. De façon préférée, la structure informatique est un serveur.

On entend par « **couplé** », au sens de l'invention, connecté, directement ou indirectement avec un ou plusieurs éléments intermédiaires. Deux éléments peuvent être couplés mécaniquement, électriquement ou liés par un canal de communication.

Dans cette description et même avant, les mêmes références sont utilisées pour désigner les mêmes éléments.

Les inventeurs ont testé plusieurs procédés et systèmes pour étudier le déplacement d'une entité mobile telle que la démarche d'un utilisateur (e.g. position, posture et/ou mouvement). Ils ont privilégié les systèmes intégrant des capteurs de mouvement, de préférence des centrales inertielles, systèmes les plus à même de fournir une réponse rapide et à un coût supportable pour une industrialisation. En effet, les procédés existants se basent généralement sur des capteurs de pression ou sur une pluralité de capteurs répartis dans la chaussure et/ou semelle ou même sur la cheville. Une telle répartition des capteurs entraine une réduction de la robustesse du système et/ou une augmentation de son coût.

Cependant, comme cela a été mentionné, la grande majorité des systèmes d'étude du déplacement d'une entité mobile telle que de la démarche d'un individu souffrent de l'autonomie de la batterie utilisée, cela étant principalement causé par la transmission en temps réel de données brutes générées par les capteurs de mouvement et en particulier par des centrales inertielles. L'alternative, à savoir dimensionner une batterie capable de tenir plusieurs dizaines d'heures concerne alors des systèmes qui ne pourraient plus être considérés comme portables ou d'usage courant.

Face à ces insuffisances, la demanderesse a développé un procédé embarquable capable de traiter les données brutes générées par les capteurs de mouvement quasi instantanément et de les encoder en un format permettant une transmission avec une consommation d'énergie réduite et/ou une mémoire de stockage réduite. En outre, le procédé permet un encodage tel que les données transmises, bien que ne nécessitant qu'une faible quantité d'énergie, conservent un maximum d'informations et permettent ensuite une analyse fine du déplacement de l'entité mobile ou de la démarche de l'utilisateur par des capteurs de mouvement.

En particulier, la demanderesse a développé une solution d'encodage de données générées par un ou plusieurs capteurs de mouvement. Comme cela a été mentionné, cet encodage pourrait être considéré comme reposant sur une phonémisation du mouvement lié au déplacement d'une entité mobile, en particulier à la démarche d'un individu (e.g. position, posture et/ou mouvement).

Une telle solution pourra s'appliquer à tout type d'entité mobile et ne saurait se limiter aux exemples présentés, dans la suite de la description, uniquement à titre illustratifs. Comme déjà évoqué, la solution d'encodage pourra s'appliquer à une entité mobile lorsque celle-ci correspond à un véhicule, un aéronef ou encore un robot, notamment un robot industriel automatisé (par exemple un bras robotisé). De façon préférée, la solution d'encodage est particulièrement adaptée aux systèmes embarqués alimentés par des batteries.

Selon un premier aspect, l'invention porte sur un procédé 1 d'encodage de données générées par un ou plusieurs capteurs de mouvement. En particulier, un tel procédé peut être mis en oeuvre pour un ou plusieurs capteurs de mouvement 21 couplés à une entité mobile, telle qu'une chaussure, notamment une chaussure portée par un utilisateur. Il pourra notamment être mis en oeuvre par un ou plusieurs processeurs 22.

Ainsi, afin d'illustrer la solution proposée par la demanderesse, dans les différents exemples ou modes de réalisation présentés dans la suite de la description, l'entité mobile considérée pourra correspondre à un être vivant comme un être humain (ci-après dénommé « utilisateur ») et le dispositif d'encodage sera présenté comme couplé, ou intégré, à un objet porté par l'entité mobile tel qu'un vêtement, une montre, un bracelet ou encore un téléphone portable. L'entité mobile peut aussi correspondre à un objet motorisé capable de se déplacer de façon autonome ou par commandes.

En particulier, un tel procédé est mis en oeuvre à partir de données générées par un ou plusieurs capteurs de mouvement 21 d'un objet tel qu'une montre 11 ou une semelle 13, 14. Par la suite, l'invention sera décrite dans une mise en oeuvre pouvant impliquer une montre ou un bracelet mais l'invention peut être mise en oeuvre pour toute entité mobile comportant un ou plusieurs capteurs de mouvement ou pour tout objet porté par une entité mobile. Certains des capteurs de mouvement 21 peuvent être répartis dans une montre 11, dans des semelles 13,14 ou bien aussi être positionnés sur des chaussures de façon à suivre les mouvements d'un utilisateur, comme son bras ou les semelles de ses articles chaussants. Les capteurs de mouvement peuvent aussi être tous intégrés dans un premier dispositif d'encodage 20 associé à la montre 11 ou à un premier bracelet et un deuxième dispositif d'encodage 20 associé à un deuxième bracelet positionné sur l'autre bras de l'utilisateur. Les capteurs de mouvement peuvent aussi être tous intégrés dans un premier dispositif d'encodage 20 associé à une première chaussure et un deuxième dispositif d'encodage 20 associé à une deuxième chaussure de la même paire. L'invention peut également s'appliquer à des données générées par des centrales inertielles couplées à des vêtements ou encore des textiles connectés, agencés pour détecter des mouvements d'un porteur dudit vêtement.

Un procédé 1 d'encodage selon l'invention permettra avantageusement une identification d'une position ou d'un mouvement d'une entité mobile telle qu'une chaussure couplée à un ou plusieurs capteurs de mouvement 21 sur une séquence temporelle ST, et notamment du bras de l'utilisateur lorsque la montre 11 et/ou le bracelet 12 est (sont) porté(s) par l'utilisateur mais aussi lorsqu'une telle chaussure est portée par un utilisateur.

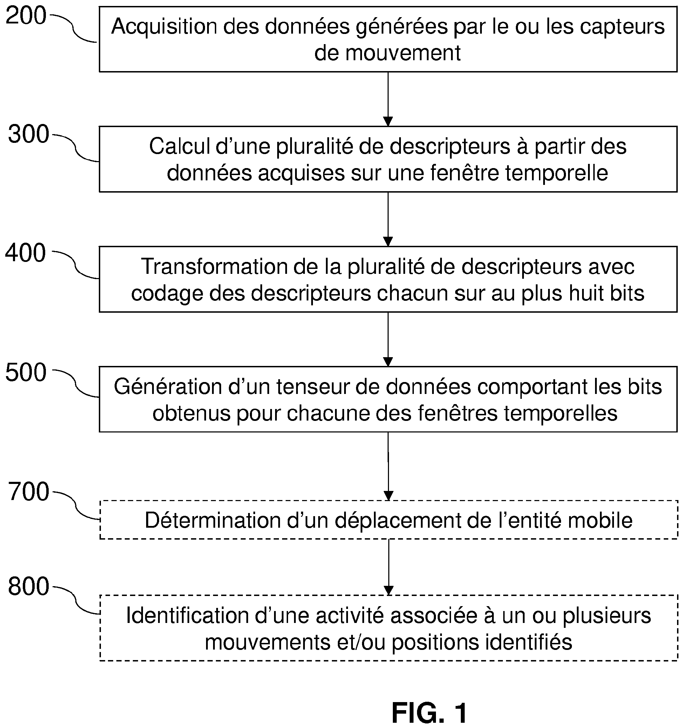

Comme cela est illustré à la figure 1, le procédé 1 d'encodage comporte : une étape d'acquisition 200 de données générées par le ou les capteurs de mouvement 21, une étape de calcul 300 d'une pluralité de descripteurs à partir des données acquises sur une fenêtre temporelle 31, une étape de transformation 400 de la pluralité de descripteurs et une étape de génération 500 d'un tenseur de données comportant les bits obtenus pour chacune des fenêtres temporelles 31, de sorte que le tenseur de données comporte un encodage d'une combinaison de plusieurs phonèmes de mouvement qui est représentative d'une position ou d'un mouvement du bras de l'utilisateur ou d'une position ou d'un mouvement de la montre 11, la chaussure ou du bracelet 12 porté par l'utilisateur.

En outre, un procédé selon l'invention peut comporter des étapes avantageuses telles que : un apprentissage 100, une calibration 210 d'un déplacement de l'entité mobile telle que la calibration de la démarche de l'utilisateur, un prétraitement 220 des données générées par les capteurs de mouvement et/ou des valeurs calculées dans le cadre du procédé, une extraction 230 d'une séquence temporelle correspondant à une unité de mouvement, une transmission 600 d'un tenseur de données, une détermination 700 d'un déplacement de l'entité mobile (e.g. l'utilisateur, une semelle) ou encore une mémorisation 900 des données. En particulier, la détermination 700 d'un déplacement de l'entité mobile peut correspondre à la détermination de la démarche d'un utilisateur.

Dans la suite de la présente description, la solution proposée par la demanderesse sera décrite dans un mode de réalisation dans lequel, le ou les capteurs de mouvement 21 comportent au moins une centrale inertielle d'au moins six axes et ce ou ces capteurs de mouvement 21 sont intégrés à une montre 11 et/ou à un bracelet 12. Elle sera aussi décrite dans un mode de réalisation dans lequel ce ou ces capteurs de mouvement 21 peuvent aussi être intégrés à une ou plusieurs semelles 13,14. La ou les semelles impliquées dans la présente invention pourront être ou non amovible(s). De façon préférée, la ou les semelles impliquées dans la présente invention est/sont amovible(s).

Néanmoins, la présente invention dans ces différents modes de réalisation, préférés ou non, pourrait être appliquée dans le cas où les capteurs de mouvement ne sont pas intégrés à une montre, à un bracelet ou une semelle. Elle pourrait en particulier être appliquée lorsque les capteurs de mouvement sont positionnés sur une chaussure, mais aussi au niveau du corps d'un utilisateur, par exemple intégrés dans un bracelet ou bien dans un vêtement porté par ce-dernier. En effet, les données de mouvements générées par un ou plusieurs capteurs de mouvement positionnés sur le corps d'un utilisateur par exemple au niveau de membres inférieurs ou de la colonne vertébrale pourraient être encodées par un procédé d'encodage selon l'invention et ou utilisées pour déterminer le déplacement d'une entité mobile tel que la démarche de l'utilisateur.

Les capteurs de mouvement pourraient également être des capteurs piézoélectriques, des fibres textiles ou des capteurs de pression qui, s'ils sont positionnés de façon adéquate, peuvent renseigner sur les mouvements d'une entité mobile notamment de l'utilisateur.

Dans le cadre de la description qui suit, de façon préférée, les un ou plusieurs processeurs sont positionnés dans une montre et/ou un bracelet. Alternativement, les un ou plusieurs processeurs pourraient être positionnés dans un boitier agencé pour être fixé à une chaussure ou à un utilisateur. Comme cela sera décrit et illustré, les un ou plusieurs processeurs pourraient être positionnés dans une semelle, par exemple au niveau d'un boitier électronique.

En particulier, les un ou plusieurs capteurs de mouvement ainsi que les un ou plusieurs processeurs sont intégrés dans un boitier électronique, tel qu'un dispositif d'encodage 20, de préférence positionné dans par exemple dans un accessoire d'un utilisateur tel qu'un vêtement, une montre, dans un bracelet ou encore dans une semelle.

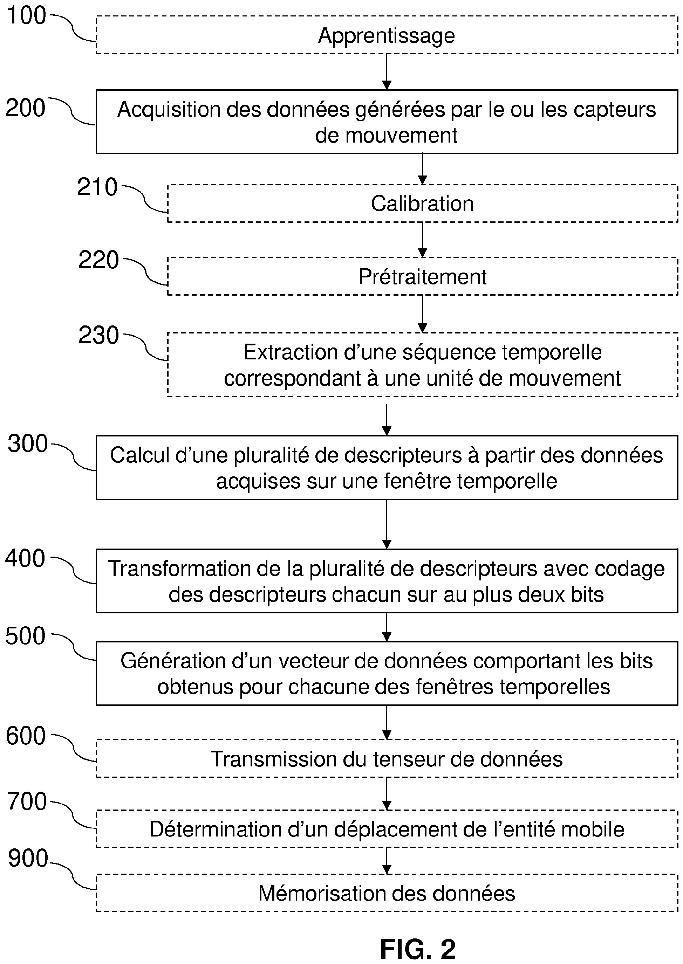

Comme illustré à la figure 2, un procédé selon l'invention peut comporter une étape 100 d'apprentissage.

Une étape 100 d'apprentissage pourra comporter une étape de collecte des données d'entrainement puis une agrégation et un stockage de ces données cibles dans une base de données. Ces données d'entrainement peuvent être ou non annotées et peuvent être utilisées pour générer des tenseurs de données utilisables dans une étape d'entrainement, de préférence distribuée et parallélisée.

De façon préférée l'étape 100 d'apprentissage pourra comporter la génération et la sélection de multiples modèles d'apprentissage par validation croisée et sélection d'hyper paramètres optimisés. Enfin, l'étape 100 d'apprentissage peut comporter une sélection du modèle d'apprentissage fournissant les meilleures performances de classification, c'est-à-dire une performance optimale et homogène entre les classes.

Une telle étape peut notamment comporter la définition d'une pluralité de valeurs de référence d'activité d'un utilisateur. Par exemple, lors de la répétition de mouvements, positions, postures et démarches effectuées par un utilisateur sur une durée déterminée, des valeurs de référence d'activité peuvent être enregistrées et classées selon une pluralité de valeurs, incluant des motifs par exemple de type statique ou dynamique.

Un procédé selon l'invention comporte une étape d'acquisition 200 de données générées par le ou les capteurs de mouvement 21, de préférence couplé(s) à une entité mobile, notamment à une montre 11 et/ou un bracelet 12 porté par un utilisateur ou encore une chaussure. L'acquisition, comme cela peut être compris à la lecture de la présente description, est de préférence réalisée pour chacun des accessoires porté par l'utilisateur et le cas échéant à la montre 11 et/ou au bracelet 12. En particulier, elle peut être réalisée pour chacune des deux chaussures et le cas échéant des deux semelles 13,14 des chaussures. Cette étape peut correspondre en particulier dans le cadre de l'invention à l'acquisition de données générées par un ou plusieurs capteurs de mouvement d'un dispositif d'encodage 20 ou de plusieurs dispositifs d'encodage 20 par exemple chacun intégré à une montre 11, à un bracelet 12 et/ou à une semelle amovible non. Cette acquisition pourra par exemple correspondre à une mise en mémoire, de préférence dans une mémoire vive des données générées par les capteurs. Cette acquisition pourra également correspondre à un chargement dans la mémoire d'un ou plusieurs processeurs destiné(s) à les traiter.

En particulier, les données acquises sont des données brutes générées par le ou les capteurs de mouvement 21. Alternativement, les données acquises peuvent aussi correspondre à des données prétraitées. Comme cela sera décrit après, les données acquises prétraitées peuvent par exemple correspondre à des données qui ont été normalisées, filtrées, complétées ou encore à des données ayant été fusionnées par exemple par un filtrage de Kalman. De façon préférée, les données acquises comportent des valeurs variables sous forme de séries temporelles. Ces valeurs variables pourront de préférence correspondre à des valeurs d'accélération et de vitesse angulaire, mais elles peuvent par exemple aussi correspondre à des valeurs générées par des capteurs de pression. Elles pourront éventuellement comporter des données de champs magnétiques ou une fusion de ces données. Ainsi, les données générées par le ou les capteurs de mouvement peuvent comporter en outre des données de champ magnétique. Les données acquises pourront être codées sur 8, 16, 32, 64, 128 bits.

Cette étape d'acquisition peut également comporter l'acquisition d'autres données telles que des données de géolocalisation, des données de température, ou encore des données de pression.

En particulier, lorsque les données acquises ont été générées par des capteurs de mouvement couplés à une entité mobile, notamment positionnés dans une montre, un bracelet ou une semelle, ces données correspondront de préférence à des valeurs d'accélération et de vitesse angulaire de l'entité mobile, notamment de la montre, du bracelet ou de la semelle.

En particulier, un procédé selon l'invention peut comprendre une étape de calibration 210. Comme cela sera décrit par la suite, les capteurs de mouvement 21 peuvent être associés à l'entité mobile, plus particulièrement à un accessoire tel qu'une montre, un bracelet, une chaussure et peuvent être intégrés dans un dispositif d'encodage 20.

Un procédé selon l'invention peut également comporter un prétraitement 220 des données générées par les capteurs de mouvement avant ou après leur acquisition.

En particulier cette étape de prétraitement 220 peut correspondre au prétraitement des valeurs d'accélération, de vitesse angulaire et/ou d'orientation d'une montre, d'un bracelet ou d'une semelle ou encore de valeur de pression. Par exemple elle peut comporter en particulier au moins un traitement sélectionné parmi : un filtrage fréquentiel, une suppression de la gravité sur les valeurs d'accélération, une suppression de la gravité, une suppression du bruit sur les valeurs d'accélération et une suppression de la dérive sur les valeurs de vitesse angulaire et/ou d'orientation de la montre, du bracelet ou de la semelle.

Comme cela a été mentionné et illustré à la figure 2, un procédé selon l'invention peut comporter une étape d'extraction 230 d'une séquence temporelle pouvant correspondre à une unité de mouvement.

L'unité de mouvement au sens de l'invention peut correspondre à un enchaînement d'un ou de plusieurs mouvements identiques ou différents, tel qu'à titre d'exemples non limitatifs une flexion, une rotation, une translation ou une extension. Ces mouvements peuvent être définis comme la période de temps allant de l'initiation d'un mouvement, par exemple du bras de l'utilisateur équipé de la montre 11 ou du bracelet 12, à l'occurrence suivante du même événement ou du même mouvement avec le même bras. L'unité de mouvement au sens de l'invention peut correspondre à un cycle de marche, un cycle de course ou encore un cycle de montée d'escalier. Ces cycles peuvent être définis comme la période de temps allant du contact initial d'un pied à l'occurrence suivante du même événement avec le même pied.

Dans le cadre de l'invention, de nombreuses méthodes de partitionnement peuvent être utilisées. Ainsi cette étape peut par exemple comporter la mise en oeuvre d'étapes classiques d'étude du mouvement (e.g. du pas), par exemple la méthode Pan-Tompkins ou la détection de seuils, de maxima et/ou de minima.

L'extraction 230 pourra avantageusement mettre en oeuvre un modèle d'apprentissage automatique, de préférence un modèle d'apprentissage automatique non supervisé.

Un procédé selon l'invention comporte également une étape de calcul 300 d'une pluralité de descripteurs à partir des données acquises sur une fenêtre temporelle 31.

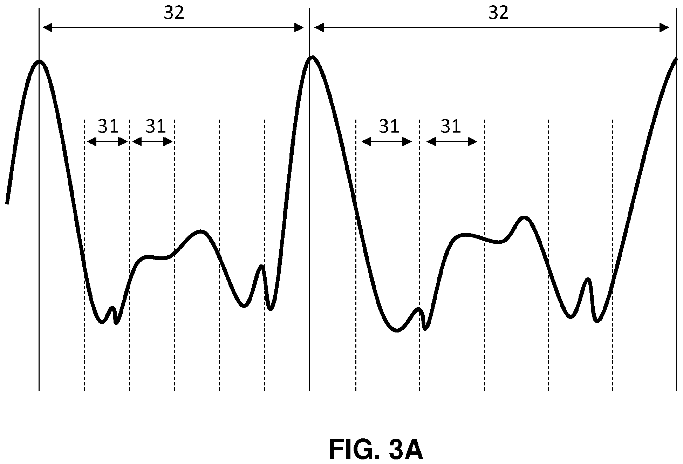

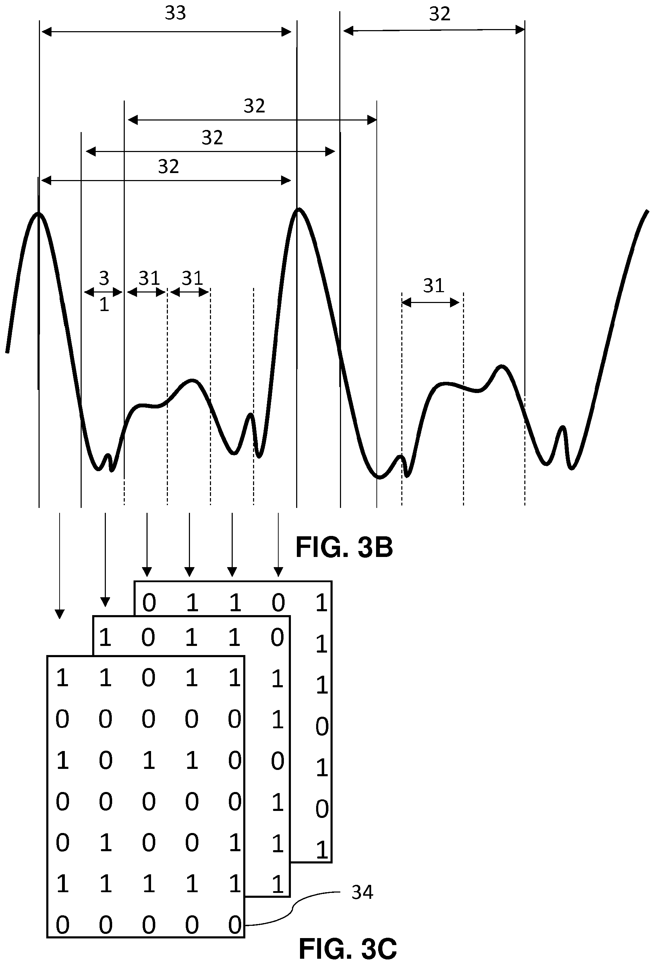

Avantageusement, cette étape de calcul peut être répétée pour plusieurs fenêtres temporelles 31 consécutives. Comme cela sera décrit par la suite, les fenêtres temporelles 31 consécutives forment une séquence temporelle 32. Cela est notamment illustré aux figures 3A et 3B.

Les figures 3A et 3B représentent des données générées par un capteur de mouvement, par exemple des données d'accélération selon l'axe x, prétraitées ou non. Ces données peuvent être divisées en séquence temporelle 32, deux à la figure 3A, puis subdivisées en fenêtres temporelles 31.

Comme cela a été présenté, un procédé selon l'invention peut comporter une étape d'extraction 230 d'une séquence temporelle 32 pouvant correspondre à une unité de mouvement. Alternativement, une séquence temporelle 32 pourra correspondre à une fenêtre glissante comme cela est illustré à la figure 3B. En outre, comme cela est illustré à la figure 3B, de façon préférée, les séquences temporelles 32 peuvent être chevauchantes.

La fenêtre temporelle 31 ne correspond pas obligatoirement à une durée prédéterminée. En outre, de préférence, elle ne correspond pas à une durée prédéterminée. Par exemple, la durée de la fenêtre temporelle 31 peut être définie une fois qu'une unité de mouvement, ou un type de mouvement a été identifié.

De façon préférée, le procédé est configuré pour traiter deux séquences temporelles 32 consécutives n'ayant pas la même durée. De façon préférée, une séquence temporelle 32, est découpée en plusieurs fenêtres temporelles 31 et dans une même séquence temporelle 32 toutes les fenêtres temporelles 31 auront la même taille. Toutefois, lorsque deux séquences temporelles 32 consécutives n'ont pas la même durée alors les fenêtres temporelles 31 d'une des deux séquence temporelle 32 n'auront pas la même durée que les fenêtres temporelles 31 de l'autre séquence temporelle 32.

Comme cela est illustré à la figure 3B, toutes les fenêtres temporelles 31 d'une séquence temporelle 32 peuvent présenter une durée identique. En outre, les fenêtres temporelles 31 de deux séquences temporelles 32 consécutives peuvent présenter des durées différentes.

Ainsi, la durée d'une fenêtre temporelle peut s'adapter à la durée d'une unité de mouvement. Cela permet de considérer des déplacements indépendamment de leur temps d'exécution.

Par exemple, la durée de chacune des fenêtres temporelles 31 d'une séquence temporelle 32 pourra correspondre à une fraction prédéterminée de la durée de la séquence temporelle 32.

Typiquement, les fenêtres temporelles 31 peuvent présenter une durée supérieure à 200 ms, de préférence supérieure à 400 ms, de façon plus préférée supérieure à 500 ms, et de façon encore plus préférée supérieure à 600 ms. Les fenêtres temporelles 31 peuvent présenter une durée inférieure à 5 s, de préférence inférieure à 4 s, de façon plus préférée inférieure à 3 s, et de façon encore plus préférée inférieure à 2 s. En particulier, la durée d'une fenêtre temporelle 31 pourra par exemple être comprise entre 25 et 1000 ms tandis que la durée d'une séquence temporelle 32 pourra par exemple être comprise entre 100 et 5000 ms. Une fenêtre temporelle 31 pourra par exemple représenter entre 1/20 et 1/4 d'une séquence temporelle 32, de préférence entre 1/10 et 1/5 d'une séquence temporelle 32.

Avantageusement, les descripteurs sont calculés à partir de fonctions mathématiques appliquées aux données générées par le ou les capteurs de mouvement 21, prétraitées ou non. Les fonctions mathématiques comportent de préférence des calculs d'asymétrie statistique, de coefficient d'aplatissement, de valeurs des extremums, de comparaisons des valeurs de bord des sous-ensembles du mouvement et/ou d'un calcul de topologie de données.

En outre, les fonctions mathématiques implémentées peuvent permettre de calculer des descripteurs dits « topologiques » permettant d'extraire, de mesurer et de comparer des informations structurelles cachées au sein de données complexes. Dans ce cas, les fonctions mathématiques peuvent être adaptées pour permettre le calcul de la topologie des données via notamment la détermination d'un diagramme de persistance uni ou multidimensionnelle (comme présenté dans H. Edelsbrunner and J. Harer. Computational Topology. An Introduction. American Mathematical Society, 2009, ou encore dans H. Edelsbrunner, D. Letscher, and A. Zomorodian. Topological persistence and simplification. Disc. Compu. Geom., 2002), du graphe de Reeb (J. Tierny, A. Gyulassy, E. Simon, and V. Pascucci. Loop surgery for volu-metric meshes: Reeb graphs reduced to contour trees. IEEE Transactionson Visualization and Computer Graphics (Proc. of IEEE VIS), 2009.), ou encore du complexe de Morse-Smale (A. Gyulassy, P. T. Bremer, B. Hamann, and V. Pascucci. A practical approach to morse-smale complex computation: Scalability and generality. IEEE Transactions on Visualization and Computer Graphics (Proc. Of IEEE VIS), 2008). Bien entendu, le calcul de ces descripteurs topologiques ne se limite pas à l'utilisation des fonctions mathématiques décrites à titre d'exemples illustratifs, d'autres fonctions mathématiques peuvent être utilisées afin de déterminer d'autres types de descripteurs topologiques.

De façon préférée, les fonctions mathématiques appliquées incorporent des données provenant de plusieurs capteurs de mouvement pour générer une donnée codée sur au plus huit bits, de préférence au plus trois bits, de façon plus préférée au plus deux bits. Par exemple, dans le cas d'une centrale inertielle générant des données d'accélération sur trois axes, un descripteur codé sur au plus trois bits, de préférence au plus deux bits, pourra avoir été généré à partir des valeurs des trois axes.

En outre, les fonctions mathématiques appliquées peuvent avantageusement incorporer des données provenant d'une fenêtre temporelle antérieure pour calculer un descripteur d'une fenêtre donnée. Ainsi, les valeurs des descripteurs peuvent transcrire une évolution des valeurs générées par les capteurs de mouvement.

De façon préférée, les fonctions mathématiques appliquées peuvent incorporer également des variables ne provenant pas des capteurs de mouvements.

De façon préférée, l'étape de calcul 300 d'une pluralité de descripteurs comportera le calcul d'au moins 4 descripteurs, de préférence d'au moins six descripteurs pour chacune des fenêtres temporelles 31.

Un procédé selon l'invention comporte une étape de transformation 400 de la pluralité de descripteurs. Cette étape de transformation 400 de la pluralité de descripteurs comporte avantageusement un codage de descripteurs, chacun sur au plus huit bits, de préférence au plus cinq bits, de façon plus préférée d'au plus quatre bits, de façon encore plus préférée d'au plus trois bits, par exemple chacun sur au plus deux bits. De façon préférée, lors de l'étape de transformation 400, les descripteurs de la pluralité de descripteurs sont transformés chacun en une donnée codée sur deux bits.

Généralement, tous les descripteurs calculés feront l'objet d'un encodage. Néanmoins, il est possible que tous les descripteurs calculés ne fassent pas l'objet d'un encodage. Par exemple, le procédé selon l'invention peut comporter une étape de sélection des descripteurs qui feront l'objet d'un encodage ou non.

Généralement, cet encodage sera exclusivement associé à un descripteur. Toutefois, il est possible que plusieurs descripteurs concourent à la définition d'une valeur encodée sur au plus huit bits qui sera complémentaire à des valeurs encodées propre à un descripteur. En outre, il est possible qu'un descripteur soit impliqué dans la définition de plusieurs valeurs encodées sur au plus huit bits. Dans un mode de réalisation, lors de l'étape de transformation 400, plusieurs descripteurs pourraient être utilisés pour générer une donnée codée sur deux bits, complémentaire.

Les inventeurs ont testé plusieurs méthodes de traitement des données générées par les capteurs de mouvements. En outre, ils ont testé plusieurs méthodes permettant de réduire la dimensionnalité des données produites par ces capteurs de mouvements. Toutefois c'est la méthode selon la présente invention qui a permis d'atteindre les meilleurs résultats en termes de consommation énergétique et de compression des données tout en conservant l'information utile des données transmises pour représenter correctement le déplacement de l'entité mobile telle que la démarche de l'utilisateur.

Le tableau 1 ci-dessous représente des données normalisées des performances du système en fonction du traitement des données acquises depuis les capteurs de données avant transmission.

Tableau 1 :

| | Consommation de la batterie | Conservation de l'information utile des données | Stockage requis des données |
|---|---|---|---|
| Pas de codage | 100 | 1 | 100 |
| Codage sur un bit ; 4 descripteurs | 1 | 38,2 | 1 |
| Codage sur deux bits ; 4 descripteurs | 5,5 | 23,7 | 3,7 |
| Codage sur trois bits ; 4 descripteurs | 15,1 | 14,2 | 5,2 |
| Codage sur quatre bits ; 4 descripteurs | 21,9 | 9 | 16 |
| Codage sur un bit ; 8 descripteurs | 3,1 | 24,1 | 3,2 |
| Codage sur deux bits ; 8 descripteurs | 10,8 | 11,9 | 7,3 |
| Codage sur trois bits ; 8 descripteurs | 26,5 | 8 | 16,5 |
| Codage sur quatre bits ; 8 descripteurs | 34 | 4,9 | 29,1 |
| Codage sur huit bits ; 8 descripteurs | 59 | 2,4 | 42,6 |

Ainsi, les valeurs de consommation de la batterie correspondent à des valeurs de consommation de la batterie normalisée en fonction de la plus haute consommation (100) et de la plus basse consommation (1). Les valeurs de conservation de l'information utile des données correspondent à des valeurs normalisées en fonction d'une capacité à conserver une quantité maximale d'information (1) et d'une conservation minimale (100). En particulier, la conservation de l'information des données est estimée à travers la précision des prédictions des mouvements par un ensemble de modèles d'apprentissage automatique et supervisés sur la base des données encodées. Enfin, les valeurs de stockage requises des données correspondent au volume en termes d'espace mémoire normalisé de la valeur la plus élevée (100) à la valeur la plus basse (1).

De façon préférée, lors de l'étape de transformation 400, au moins huit descripteurs sont utilisés et chacun codé sur au moins un bit, de façon plus préférée au moins deux bits. De façon générale, lors de l'étape de transformation 400, des descripteurs sont chacun codés sur au plus huit bits, de façon préférée au plus six bits et de façon encore plus préférée au plus quatre bits. De préférence, l'encodage est un encodage numérique contrairement à l'encodage d'une tension de sortie d'une centrale inertielle qui est généralement issu d'un traitement analogique.

Par exemple, parmi les modes de réalisation préférée, lors de l'étape de transformation 400, le codage est réalisé sur :

- un bit pour entre 4 et 20 descripteurs, par exemple quatre descripteurs ou huit descripteurs ;
- deux bits pour entre 4 et 20 descripteurs, par exemple quatre descripteurs ou huit descripteurs ;
- trois bits pour entre 4 et 20 descripteurs, par exemple quatre descripteurs.

Le codage sera de préférence réalisé pour chacune des fenêtres temporelles 31.

Ainsi, les données encodées sur au plus deux bits et associés à une fenêtre temporelle 31 seront de préférence représentatives de ce qui pourrait être considéré comme un phonème de mouvement de l'entité mobile ou du bras de l'utilisateur équipé d'une montre ou d'un bracelet ou de la chaussure. Alternativement, ces données encodées sur au plus deux bits et associés à une fenêtre temporelle 31 peuvent être considérées comme un phonème si une phonémisation des mouvements associés au déplacement de l'entité mobile en particulier à la démarche est considérée.

Avantageusement, plusieurs données encodées (e.g. une pluralité de descripteurs chacun encodé sur au plus deux bits) caractérisent un phonème de mouvement et plusieurs phonèmes de mouvement caractérisent un mouvement.

Ainsi, les données encodées sous une série de valeurs encodées sur au plus deux bits associés à une fenêtre temporelle 31 sont représentatives d'un micromouvement de l'utilisateur, en particulier d'un objet tel qu'une montre ou un bracelet ou une chaussure porté par l'utilisateur et plus particulièrement d'une semelle 13,14.

Le tableau 2 ci-dessous illustre un exemple de données obtenues à la suite d'une étape de transformation selon l'invention.

| | FT1 | | FT2 | | FT3 | |
|---|---|---|---|---|---|---|
| | Calculée | Encodée | Calculée | Encodée | Calculée | Encodée |
| Descripteur 1 | 12,45989 | -1 | 70,8759 | 1 | 49,3476 | 0 |
| Descripteur 2 | 9,7228 | -1 | 23,7459 | 0 | 93,8016 | 2 |
| Descripteur 3 | 69,7904 | 1 | 69,0302 | 1 | 1,6486 | -1 |
| Descripteur 4 | 17,9573 | -1 | 24,2259 | 0 | 38,1186 | 0 |
| Descripteur 5 | 24,2259 | 0 | 147,2232 | 2 | 72,8551 | 1 |

Le codage des descripteurs et donc des valeurs obtenues peut être réalisé sur la base d'intervalles prédéterminés. Par exemple, dans le cadre du descripteur 1 pouvant correspondre à une valeur maximale d'accélération sur l'axe Y, le procédé selon l'invention peut faire appel à des règles prédéterminées selon lesquelles une valeur inférieure à 20 sera transformée en une valeur « -1 », une valeur de 20 à 50 (50 inclus) sera transformée en une valeur « 0 », une valeur comprise entre 50 (50 exclu) et 80 (80 exclu) sera transformée en une valeur « 1 », et une valeur supérieure ou égale à 80 sera transformée en une valeur « 2 ». Ainsi, le descripteur 1 est transformé en une donnée codée sur au plus huit bits, en l'occurrence 2 bits.

Les intervalles prédéterminés peuvent être modifiés dans le temps soit automatiquement en fonction des valeurs obtenues soit au travers de mises à jour.

Le codage des descripteurs peut également correspondre à des comparaisons de valeurs de descripteurs différents ou d'un même descripteur sur plusieurs fenêtres temporelles 31. Par exemple, si le descripteur 3, qui peut correspondre à la moyenne de l'accélération sur l'axe x, ne varie pas de plus de 10 % entre les fenêtres FT0 et FT1 alors sa valeur sera transformée en une valeur « 1 ». De la même façon, le descripteur 3 ne varie pas de plus de 10 % entre les fenêtres FT1 et FT2 alors sa valeur sera transformée en une valeur « 1 ». Par contre, le descripteur 3 varie de plus de 80 % à la baisse entre les fenêtres FT2 et FT3 alors sa valeur sera transformée en une valeur « -1 ».

Ainsi, le codage des descripteurs, c'est-à-dire la génération pour chacun des descripteurs d'une donnée codée sur au plus huit bits, peut être avantageusement basée sur des règles prédéterminées. En outre, elle peut avantageusement prendre en compte des intervalles prédéterminés, des évolutions de valeurs dans le temps et/ou des résultats d'opération mathématiques entre les descripteurs.

Un procédé selon l'invention pourra conduire, comme cela sera décrit par la suite, la génération d'un tenseur de données qui pourrait selon l'exemple ci-dessus être représenté sous la forme suivant :

$$V1 = \{-1,-1,1,-1,0 \,; 1,0,1,0,2 \,; 0 \,; 2,-1,0,1\}$$

Un tel tenseur de données comporte plusieurs ensembles de données encodées en au plus deux bits. Par exemple il comporte un ensemble de cinq valeurs encodées en au plus deux bits correspondant à l'encodage de descripteurs pour la fenêtre temporelle notée « FT1 » ; un ensemble de cinq valeurs encodées en au plus deux bits correspondant à l'encodage de descripteurs pour la fenêtre temporelle notée « FT2 » ; et un ensemble de cinq valeurs encodées en au plus deux bits correspondant à l'encodage de descripteurs pour la fenêtre temporelle notée « FT3 ».

Ainsi, si les fenêtres temporelles FT1, FT2 et FT3 (tableau 2) correspondent à une séquence temporelle 32 et donc à une unité de mouvement alors ledit mouvement d'une seconde peut être décrit par quinze valeurs encodées sur deux bits soit dans le cas illustré par 30 bits. En outre, dans le cas de l'utilisation d'une séquence temporelle 32 glissante alors, un tel mouvement pourrait être représenté par cinq fenêtres temporelles 32 soit 150 bits.

Cela est à comparer aux 14400 bits des données de capteurs de mouvements tels que des centrales inertielles 9 axes échantillonnant un mouvement d'une seconde à 100 Hz.

La présente invention permet donc, avec une réduction minime de l'information, une réduction des flux de données nécessaires d'un ordre cent environ engendrant une réduction de la consommation énergétique et des capacités de stockage nécessaires.

Ainsi, un procédé selon l'invention peut comporter également une étape de génération 500 d'un tenseur de données comportant les données codées obtenues pour chacune des fenêtres temporelles 31.

Le tenseur de données comportant les données codées obtenues pour chacune des fenêtres temporelles 31 notées « FT » dans le tableau 2 peut correspondre à une pluralité de tenseurs de données comportant chacun les données codées obtenues pour une fenêtre temporelle 31.

Ainsi, le tenseur de données comporte de préférence un encodage d'une combinaison de plusieurs phonèmes de mouvement de l'entité mobile et en particulier de l'utilisateur de préférence d'une montre ou d'un bracelet ou d'une chaussure et en particulier d'une semelle. Le tenseur de données comporte avantageusement un encodage qui est représentatif d'une position ou d'un mouvement de la montre ou du bracelet porté par l'utilisateur comme une chaussure portée par un utilisateur.

Un tenseur de données selon l'invention permet avantageusement d'identifier un état physique et en particulier d'un mouvement d'une entité mobile. Un mouvement pourra correspondre à une succession de positions. Si toutes les positions sont les mêmes alors il n'y a pas de mouvement. Si au moins une position est différente alors il y a eu un mouvement.

Les inventeurs ont en outre montré que l'utilisation d'un tel tenseur de données, comportant des données codées sur au plus huit bits, de préférence au plus trois bits, de façon plus préférée au plus deux bits, permet d'améliorer la performance d'un modèle d'apprentissage automatique reposant sur un tel tenseur de données. En effet, un modèle d'apprentissage automatique reposant sur un encodage selon l'invention présentera le cas échéant une taille de réseau de neurones réduite et/ou un temps de calcul réduit tout en étant tolérant aux variabilités statistiques des populations représentatives des jeux de données et flexible à la sensibilité mécanique des capteurs inertiels.

Avantageusement, le tenseur de données comporte une série codée sur quatre bits au plus, de préférence trois bits au plus et de façon plus préférée deux bits au plus. Cependant, il pourra comporter d'autres données qui ne seront pas obligatoirement codées sur au plus deux bits. Par exemple, le tenseur de données pourra comporter des données non encodées pouvant correspondre à des attributs calculés au niveau par exemple de la montre ou du bracelet ou de la semelle et décrivant un aspect de l'entité mobile (e.g. l'utilisateur) et en particulier de la démarche de l'utilisateur.

Alternativement, le tenseur de données 34 correspond à une série codée sur huit bits au plus, de préférence au plus trois bits, de préférence deux bits au plus.

L'étape de génération 500 peut comporter la génération de plusieurs tenseurs de données pour une fenêtre temporelle 31. C'est par exemple le cas si un tenseur de données est généré pour chacun des axes d'une plateforme inertielle. Alternativement un tenseur de données peut comporter les données codées concernant plusieurs axes d'une plateforme inertielle.

Un procédé selon l'invention peut comporter également une étape de transmission 600 du tenseur de données.

Avantageusement, les données étant codées sur au plus huit bits, la transmission peut avantageusement faire l'objet d'une concaténation au sein de trames de données permettant le transfert de trames de données par exemple de 8 bits, 16 bits ou 32 bits. Ainsi le nombre de message est réduit par l'optimisation de l'occupation des trames. Ainsi les trames de transmission sont optimisées réduisant le cout énergétique et le cout de vérification des paquets (e.g. Ack...). Cela est particulièrement significatif dans le cadre des transmissions basses énergie telle que BLE (pour Bluetooth Low Energy en terminologie anglo-Saxonne) ou Zigbee.

Bien qu'une étape de détermination d'un mouvement ou plus globalement d'un déplacement de l'entité mobile (e.g. l'utilisateur) d'une démarche d'un utilisateur puisse être réalisée au niveau du dispositif réalisant l'étape d'acquisition 200 de données générées par le ou les capteurs de mouvement, il est également possible de diviser les traitements et d'interpréter le tenseur de données dans un dispositif électronique différent du dispositif électronique ayant généré le tenseur de données.

La transmission pourra par exemple être réalisée par des moyens de communication sans fil tel que des radiocommunications. La transmission du tenseur de données se faisant alors de préférence vers un dispositif mobile externe. La transmission du tenseur de données pourrait également se faire vers un dispositif informatique distant tel qu'un serveur de traitement de données.

La transmission du tenseur de données peut également se faire directement vers un dispositif de traitement des données mesurées par un capteur de mouvement à un autre. Par exemple d'une montre à un bracelet ou d'un bracelet à un autre bracelet. Également, d'une semelle à une autre semelle d'une même paire de chaussures. La transmission du tenseur de données peut être réalisée en continu avec la transmission d'un tenseur de données à la fois. Elle peut également correspondre à la transmission de plusieurs tenseurs de données. Par exemple une transmission peut comporter plusieurs tenseurs de données chacun correspondant à une fenêtre temporelle différente ou encore à des tenseurs de données générés à partir de variables de capteurs de mouvement différents.

Selon un autre aspect, la présente invention porte sur un procédé de détermination 2 d'un déplacement d'une entité mobile. De façon préférée, le procédé de détermination 2 d'un déplacement d'une entité mobile correspond à un procédé de détermination de la démarche d'un être vivant, de préférence d'un être humain, utilisant le dispositif d'encodage 20.

De préférence, la détermination du déplacement de l'entité mobile ou la détermination de la démarche correspond à la détermination d'une position, un mouvement, ou une activité de l'entité mobile, ou en particulier à une position, un mouvement, ou une activité de l'utilisateur.

En particulier, la présente invention porte sur un procédé de détermination 2 de la démarche d'un utilisateur. De préférence, la démarche de l'utilisateur correspond à une posture, un mouvement, ou une activité de l'utilisateur.

Le procédé de détermination 2 sera de préférence exécuté par un ou plusieurs processeurs. Avantageusement, le procédé de détermination 2 d'un déplacement d'une entité mobile (e.g. un utilisateur) telle que la détermination 2 de la démarche d'un utilisateur est mis en oeuvre au moins en partie au niveau d'un dispositif d'encodage 20, de préférence couplé à l'utilisateur, de préférence positionné sur un bras de l'utilisateur ou dans une chaussure, par exemple dans une montre, une semelle, au niveau d'une chaussure ou dans un bracelet porté par l'utilisateur. Alternativement, le procédé de détermination 2 est mis en oeuvre au moins en partie au niveau d'un terminal externe 30 tel qu'un dispositif mobile, ou d'une infrastructure informatique.

Une telle détermination se fera de préférence à partir de données encodées, ou susceptibles d'être encodées, selon un procédé d'encodage 1 selon la présente invention.

En particulier, le procédé de détermination 2 d'un déplacement d'une entité mobile (e.g. un utilisateur) telle que la détermination 2 de la démarche selon l'invention se basera au moins en partie sur des données encodées. En particulier, les données encodées prennent la forme d'une série temporelle pour laquelle chaque fenêtre temporelle 31 comporte au moins quatre données encodées sur au plus huit bits, de préférence au plus trois bits, de façon plus préférée au plus deux bits. De façon préférée, chaque fenêtre temporelle 31 comporte entre 4 et 50 données encodées sur au plus huit bits, de préférence au plus trois bits, de façon plus préférée au plus deux bits. La série temporelle peut correspondre par exemple à une ou plusieurs unité(s) de mouvement et chacune des unités de mouvement étant formées par plusieurs fenêtres temporelles 31.

En particulier, le procédé de détermination 2 d'un déplacement d'une entité mobile (e.g. un utilisateur) telle que la détermination 2 de la démarche selon l'invention se basera au moins en partie sur des données encodées qui prennent la forme d'un ou plusieurs tenseurs de données comportant pour chaque fenêtre temporelle 31 au moins quatre données encodées sur au plus huit bits, de préférence au plus trois bits, de façon plus préférée au plus deux bits. De façon préférée, chaque fenêtre temporelle 31 est représentée par 4 à 50 données encodées sur au plus trois bits, de préférence au plus deux bits. Les un ou plusieurs tenseurs de données correspondant par exemple à un ou plusieurs mouvement(s) et chacun des mouvements étant formés représentés par plusieurs fenêtres temporelles.

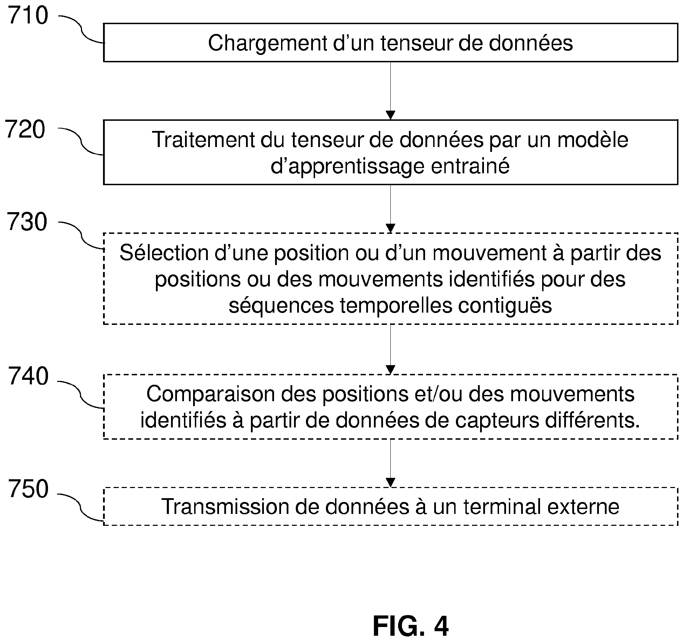

Comme cela est illustré à la figure 4, le procédé de détermination 2 d'un déplacement d'une entité mobile (e.g. un utilisateur) telle que la détermination 2 de la démarche comporte : une étape de chargement 710 d'un, de préférence de plusieurs tenseurs de données ; et une étape de traitement 720 des tenseurs de données de préférence par un modèle d'apprentissage automatique entrainé de manière à identifier une position ou un mouvement.

Le procédé de détermination 2 d'un déplacement d'une entité mobile (e.g. un utilisateur) telle que la détermination 2 de la démarche correspond de préférence à une détermination en temps réel à un ou plusieurs éléments constitutifs du déplacement d'une entité mobile, par ex. d'un utilisateur telle que de la démarche de l'utilisateur. C'est-à-dire qu'après la génération de données par un ou plusieurs capteurs de mouvement, de préférence en moins de 60 secondes, de façon plus préférée en moins de 30 secondes, de façon encore plus préférée en moins de 10 secondes, le procédé selon l'invention est en mesure de déterminer un élément du déplacement d'une entité mobile (e.g. un utilisateur) comme la démarche de l'utilisateur tel qu'une position, une posture, un pas, un saut, ou tout autre mouvement constitutif du déplacement de l'entité mobile (e.g de la démarche d'un utilisateur).

Comme illustré à la figure 4, le procédé de détermination 2 selon l'invention peut également comporter les étapes suivantes : une étape de sélection 730 d'une position, posture ou d'un mouvement pour un intervalle temporel à partir des positions, postures ou des mouvements identifiés pour des séquences temporelles contiguës, une étape de comparaison 740 de la position, de la posture et/ou du mouvement identifié à partir de données de capteurs différents et une étape de transmission 750 de données à un terminal externe.

Comme mentionné, le procédé de détermination 2 d'un déplacement d'une entité mobile (e.g. détermination de la démarche d'un utilisateur) comporte une étape de chargement 710 d'un ou de plusieurs tenseurs de données.

Le chargement du tenseur de données peut correspondre au chargement de plusieurs tenseurs de données. Cette étape peut correspondre en particulier dans le cadre de l'invention à la mise en mémoire, de préférence dans une mémoire vive d'un ou plusieurs tenseurs de données. Cette acquisition pourra également correspondre à un chargement dans la mémoire d'un ou plusieurs processeurs destiné(s) à les traiter.

Comme mentionné, le procédé de détermination 2 comporte une étape de traitement 720 du ou des tenseurs de données par un modèle d'apprentissage automatique.

En particulier, l'étape de traitement 720 du tenseur de données par un modèle d'apprentissage automatique comportera l'identification de classes d'événements à partir du ou des tenseurs de données. En particulier, l'étape de traitement 720 permet à partir de plusieurs tenseurs de données, de préférence chacun correspondant à un phonème de mouvement, d'identifier une classe d'événements. De façon préférée, l'étape de traitement 720 est réalisée sur des tenseurs de données d'ordre 3 comportant plusieurs tenseurs de données.

La figure 3C illustre par exemple trois tenseurs de données 34 sous la forme de trois tenseurs d'ordre 2 comportant chacun cinq tenseurs de données. Chaque tenseur de données correspond ici à des données calculées à partir d'une fenêtre temporelle 31 et un tenseur de données 34 peut correspondre alors à une séquence temporelle 32. Un tenseur d'ordre 2 illustré à la figure 3C comporte des données codées sur un bit (0 ou 1) pour sept descripteurs et sur cinq fenêtres temporelles 31.

À la figure 3C, les tenseurs 34 correspondent à des fenêtres temporelles 32 glissantes et chevauchantes. Une séquence temporelle 32 pourrait également correspondre à une fenêtre dont la taille varie au fur et à mesure des calculs de position, posture, mouvement ou plus largement classe d'événement. Par exemple, une première séquence temporelle 32 comporte trois fenêtres temporelles 31, une seconde séquence temporelle 32 comporte quatre fenêtres temporelles 31 et ainsi de suite jusqu'à atteindre une valeur prédéterminée de dimension maximale de séquence temporelle 32. La séquence temporelle 32 pourrait alors voir sa taille réduite à une valeur prédéterminée de dimension minimale.

Ainsi, les données codées sous bits associés à une ou plusieurs fenêtres temporelles 31, de préférence représentatifs d'un phonème de mouvement, seront traitées par un modèle d'apprentissage automatique pour inférer une classe d'événement telle qu'une classe de mouvement, de position ou de posture. Une classe de mouvement peut correspondre au sens de l'invention à un mouvement se déroulant sur une durée très courte, par exemple moins de 500 ms et peuvent correspondre à des portions de mouvement plus long. Par exemple, il sera possible dans le cadre de l'invention, à partir des bits associés à une fenêtre temporelle 31 ou à une séquence temporelle 32, de déterminer s'il y a un agenouillement en cours, un pas avant, un pas arrière, etc.

Avantageusement, la solution selon l'invention utilise un ou plusieurs algorithmes issus d'apprentissages automatiques, de préférence préalablement calibrés, pour le traitement du ou des tenseurs de données. Ces algorithmes peuvent avoir été construits à partir de différents modèles d'apprentissage, supervisés, non supervisés ou par renforcement. Un algorithme d'apprentissage non supervisé peut par exemple être sélectionné parmi un modèle de mélange Gaussien non supervisé, une classification ascendante hiérarchique (Hierarchical clustering Agglomerative en terminologie anglo-Saxonne), une classification descendante hiérarchique (Hierarchical clustering divisive en terminologie anglo-Saxonne). Alternativement, l'algorithme repose sur un modèle d'apprentissage statistique supervisé configuré de façon à minimiser un risque de la règle d'ordonnancement et ainsi permettant d'obtenir des règles de prédiction plus performantes. Dans ce cas, les étapes de calcul de détermination et d'estimations peuvent être basées sur un modèle, entrainé sur un jeu de données et configuré pour prédire une étiquette (e.g. déplacement/démarche similaire ou non au déplacement / à la démarche enregistrée). Par exemple, aux fins de la calibration, il est possible d'utiliser un jeu de données représentatif d'une situation dont l'étiquette est connue. Le jeu de données peut également comprendre des étiquettes multiples. L'algorithme peut être issu de l'utilisation d'un modèle d'apprentissage statistique supervisé sélectionné par exemple parmi les méthodes à noyau (e.g. Séparateurs à Vaste Marge - Support Vector Machines SVM, Régression à noyau de type Ridge), les méthodes d'ensembles (e.g. arbres de décision, Forêts aléatoires), partitionnement hiérarchique, partitionnement en k-moyenne, régression logique ou les réseaux de neurones.

De façon préférée, le modèle d'apprentissage automatique entraîné est de préférence sélectionné parmi un réseau de neurones, par exemple un modèle d'apprentissage profond, un réseau de neurones récurrent (e.g. un LSTM, pour « Long Short-Term Memory » en terminologie anglo-saxonne), un perceptron multicouche ou encore d'autres méthodes d'apprentissage automatique telles que des méthodes à base d'arbres décisionnels (e.g. forêt aléatoire) ou des méthodes de clustering (e.g. Méthode par partitionnement ou Méthodes hiérarchiques).

En outre, avantageusement, le procédé selon l'invention pourra comporter une étape de sélection 730 d'une position, d'une posture ou d'un mouvement à partir des postures ou des mouvements identifiés pour des séquences temporelles 32 contiguës. Cette position, posture ou ce mouvement sélectionné peut en particulier correspondre à la position à la posture ou au mouvement d'un utilisateur durant un intervalle temporel 33 particulier ayant fait l'objet de plusieurs séquences temporelles 32 contiguës comme cela est illustré à la figure 3B. L'intervalle temporel 33 peut correspondre à une unité de mouvement.

Ainsi, l'étape de sélection 730 d'une position, d'une posture ou d'un mouvement peut être considérée comme une validation d'un mouvement, d'une position ou d'une posture à partir du traitement de plusieurs classes de mouvement, de position ou de posture inférés lors de l'étape de traitement 720 du tenseur de données par un modèle d'apprentissage automatique.

L'étape de sélection 730 pourrait comporter une comparaison de la position, de la posture ou du mouvement identifié pour des séquences temporelles 32 contiguës. En effet, en particulier lorsqu'une fenêtre glissante est utilisée, ou plus généralement lorsqu'un mouvement est représenté par plusieurs séquences temporelles contiguës, comme c'est le cas à la figure 3B, le procédé selon l'invention peut comporter une identification d'une posture, d'une posture ou d'un mouvement pour chacune des plusieurs séquences temporelles 32 contiguës. Puis, le procédé comporte une sélection de la position, de la posture ou du mouvement le plus représenté au sein des séquences temporelles 32 contiguës étudiées. Cela permet de réduire les risques de faux positifs en tirant parti de la reconnaissance en temps réel de position, de posture ou de mouvement au sein de séries temporelles et d'une validation multiple d'une même position, posture ou mouvement. Par exemple, une position, une posture ou un mouvement peut être sélectionné à partir d'une analyse d'au moins 3, de préférence d'au moins 4 et de façon encore plus préférée d'au moins cinq séquences temporelles contiguës.

De façon préférée, l'étape de sélection 730 est réalisée sur des tenseurs de données d'ordre 3 comportant plusieurs tenseurs de données.

En outre, avantageusement, le procédé selon l'invention pourra également comporter une étape de comparaison 740 de position, de postures et/ou de mouvement identifiés, par exemple pour un instant donné, à partir de données de capteurs différents.

Une telle comparaison 740 de position, de postures et/ou de mouvement identifiés peut être utilisée pour déterminer le déplacement de l'entité mobile, par ex. la démarche de l'utilisateur par l'association d'informations provenant de plusieurs capteurs.

Cette comparaison 740 se fait de façon préférée pour des intervalles de temps synchronisés de façon que les données mesurées et interprétées correspondent bien à un même moment du déplacement de l'entité mobile, par ex. la démarche de l'utilisateur. Une telle synchronisation est possible par exemple grâce à l'utilisation d'une horloge synchronisée ou encore à des communications inter dispositifs.

En particulier, le procédé de détermination 2 peut être mis en oeuvre à partir de tenseurs de données provenant de plusieurs sources. Par exemple, de plusieurs capteurs de mouvement positionnés en différents points d'une entité mobile (e.g. d'un utilisateur) générant eux aussi des tenseurs de données selon la présente invention.

Dans ces conditions, le procédé de détermination 2 peut avantageusement comporter, pour un intervalle de temps donné, une comparaison 740 de la position, de la posture, et/ou du mouvement, identifié à partir des données d'un premier capteur avec la position, la posture, ou le mouvement, identifié à partir d'un second capteur. Par exemple, l'étape de comparaison 740 de la position, de la posture, et/ou du mouvement, identifié peut comporter une comparaison de la position, de la posture, ou du mouvement identifié à partir d'un capteur positionné sur le bras droit d'un utilisateur avec la position ou le mouvement identifié à partir d'un capteur positionné sur le bras gauche de l'utilisateur. En particulier, l'étape de comparaison 740 de la posture, et/ou du mouvement identifié peut comporter une comparaison de la posture, ou du mouvement identifié à partir d'un capteur positionné sur le pied droit avec la posture ou le mouvement identifié à partir d'un capteur positionné sur le pied gauche.

L'étape de comparaison 740 de positions, de postures et/ou de mouvements identifiés pour un instant donné pour des capteurs différents pourra mettre en oeuvre des positions, des postures ou des mouvements identifiés à partir de données de différents capteurs ne mettant pas en oeuvre le tenseur de données selon l'invention. Par exemple, la position, la posture, ou le mouvement, identifié à partir de données encodées selon l'invention pourra être comparé avec la position, la posture, ou le mouvement, identifié avec d'autres méthodes.

En particulier, le procédé de détermination 2 peut avantageusement comporter une comparaison de la position, de la posture ou du mouvement identifié pour chacun des bras et/ou des pieds d'un utilisateur à un référentiel. Une telle comparaison permettra de renforcer la spécificité de la performance de détermination et dans certains cas de déterminer un type de mouvement ou une posture complexe. En effet, certains mouvements ou postures ne pourront être déterminés avec exactitude qu'avec la prise en compte des résultats de plusieurs capteurs de mouvement. Le référentiel pourra consister en une base de données, un algorithme transcrivant des règles expertes, ou encore un modèle d'apprentissage automatique.

De préférence, le procédé peut également comporter une étape de transmission 750 de données à un terminal externe 30. Cette transmission se fait de préférence ponctuellement. En particulier cela peut correspondre à la transmission de l'ensemble des données générées et/ou calculées à un terminal externe 30.

En outre, le procédé peut comporter la transmission des données reçues par le terminal externe 30 à un ou d'autres terminaux externes qui pourraient être impliqués dans le calcul d'autres paramètres du déplacement de l'entité mobile (e.g. de la démarche de l'utilisateur) ou dans leur affichage.

Les données transmises peuvent par exemple être des données brutes telles qu'elles ont été générées par les capteurs de mouvement, des données prétraitées, des données transformées ou encore des données de déplacements (e.g. de démarches).

En outre, un procédé de détermination 2 selon l'invention peut comporter également une étape d'identification 800 d'une activité associée à un ou plusieurs mouvements, positions et/ou postures identifiés.

Cette identification d'une activité comporte de préférence la comparaison de valeurs de référence d'activité aux valeurs d'un ou de plusieurs tenseurs de données ou aux valeurs d'un ou plusieurs mouvements, positions ou postures identifiés.

Il est alors possible, de préférence à partir de plusieurs mouvements et/ou positions identifiés de déterminer quel est, d'une part, le type d'entité mobile dont les mouvements/positions ont été identifiées et d'autre part quelle est l'activité pratiquée par l'entité mobile. En effet, l'homme du métier comprendra qu'en fonction du type d'entité mobile considérée, les mouvements et positions identifiés seront radicalement différents et permettront de caractériser l'entité mobile. En outre, lorsque l'entité mobile est par exemple un utilisateur, il sera possible de déterminer le type d'activité pratiquée par ce-dernier en fonction des mouvements/positions identifiés. Il est alors possible, de préférence à partir de plusieurs mouvements et/ou postures identifiés de déterminer quelle est l'activité pratiquée par l'utilisateur.

Ci-après, les différentes étapes obligatoires ou non d'un procédé de détermination 2 d'un déplacement d'une entité mobile (e.g. de la démarche d'un utilisateur) sont présentées pour résumer et mettre en perspective la présente invention.

Lors du traitement 720 du tenseur de données par un modèle d'apprentissage automatique, la présente invention pourra par exemple étudier une pluralité de fenêtres temporelles 31 de façon à identifier une classe de mouvement, telle qu'un pas, pour chacune des fenêtres temporelles 31. De façon plus préférée, lors du traitement 720 du tenseur de données en particulier d'un tenseur de données d'ordre 3 par un modèle d'apprentissage automatique, la présente invention pourra étudier une pluralité de fenêtres temporelles 31 de façon à identifier une classe de mouvement, telle qu'un pas, pour chacune des séquences temporelles 32 comportant plusieurs fenêtres temporelles 31.

Une étape complémentaire optionnelle de sélection 730 d'une posture ou d'un mouvement peut être mise en oeuvre de façon à confirmer, par l'analyse des classes de mouvement identifiées pour plusieurs fenêtres temporelles 31 contiguës ou pour plusieurs séquences temporelles 32, la présence d'un mouvement particulier tel qu'une flexion, translation, rotation, extension, pas. Par exemple, une séquence temporelle 32 peut indiquer un pas alors que les trois suivantes indiquent une glissade. Dans ce cas, au terme de l'étape complémentaire de sélection 730 d'une posture ou d'un mouvement, la présente invention pourra indiquer la survenue d'une glissade.

En outre, de manière plus générale, l'analyse des classes de mouvement identifiées pour plusieurs fenêtres temporelles 31 contiguës ou pour plusieurs séquences temporelles 32, peut permettre d'indiquer la survenue d'un mouvement à « risque ». En effet, lorsque le mouvement identifié n'est associé à aucune classe de mouvement ou de posture inféré, celui-ci peut être la conséquence d'un mouvement involontaire de l'utilisateur et potentiellement source d'une lésion musculaire et/ou squelettique pour l'utilisateur. Cela peut être en particulier applicable dans des métiers physiquement éprouvant pouvant conduire à des blessures notamment au niveau des membres inférieurs tels que les métiers du bâtiment et des travaux publics.

Dans une étape complémentaire optionnelle de comparaison 740 de postures et/ou de mouvement identifiés, la présente invention étudiera des données générées par d'autres capteurs, de préférence également sous la forme de tenseur de données (e.g. un tenseur de données d'ordre 3), par exemple un tenseur de données généré à partir des mouvements du pied gauche. Dans la suite de l'exemple ci-dessus, l'analyse du ou des tenseurs de données provenant du pied gauche peut indiquer une flexion du genou. Dans ce cas, le mouvement identifié au terme de l'étape 740 pourra par exemple être représentatif d'un mouvement de course de l'utilisateur ou encore d'un mouvement de protection en boxe ou encore d'un fauchage. Ainsi, le mouvement ou la posture identifié(e) au terme de l'étape 740 peut être considéré comme un mouvement plus complexe. En effet, les tenseurs de données, plus particulièrement les tenseurs de niveau 3, peuvent avantageusement être associés à un motif de déplacement particulier décrivant un ou plusieurs mouvements spécifiques.

Enfin, dans une étape d'identification de l'activité 800, la présente invention pourra à partir de données générées déterminer plusieurs mouvements qui conduiront par exemple à identifier que l'activité réalisée est une activité sportive de type judo, course ou boxe.

De préférence, le procédé peut également comporter une étape de mémorisation 900 de données. Cette mémorisation se fait de préférence en continu. En particulier cela peut correspondre à la mémorisation de l'ensemble des données générées et/ou calculées dans le cadre d'un procédé selon l'invention. Les données mémorisées peuvent par exemple être des données brutes telles qu'elles ont été générées par les capteurs de mouvement, des données prétraitées, des données transformées ou encore des données de déplacement (e.g. démarche). De préférence, les données mémorisées correspondent à des données transformées ou encore des données de déplacement (e.g. démarche).

Avantageusement, par opposition aux données brutes et/ou prétraitées qui sont sauvegardées sur une durée courte (p. ex. inférieure à 5 minutes) par exemple sur une mémoire cache, une valeur de tenseur de données ou de déplacement (e.g. démarche) est mémorisée à plus long terme, par exemple sur une mémoire.

Avantageusement, le calcul d'un tenseur de données et/ou d'un déplacement (e.g. d'une démarche) déterminée est fait en temps réel, c'est-à-dire moins de 1 heure après la génération des données par l'un ou plusieurs des capteurs de mouvement couplé à l'entité mobile (e.g. d'une semelle), de préférence moins de 10 minutes, de façon plus préférée moins d'une minute, de façon encore plus préférée moins de 10 secondes.

**Selon un autre aspect,** l'invention porte sur un **dispositif d'encodage 20 de données générées** par un ou plusieurs capteurs de mouvement 21, de préférence couplés à une entité mobile, par exemple à une montre ou un bracelet ou une chaussure portés par un utilisateur.

Un tel dispositif d'encodage 20 de données peut en particulier être utilisé pour identifier une position ou un mouvement d'une entité mobile, par ex. d'un utilisateur sur une séquence temporelle 32, notamment lorsque la chaussure est portée par un utilisateur. Plus largement, s'il est appliqué à des capteurs de mouvement 21 positionnés sur le corps d'un utilisateur, il peut être utilisé pour identifier une position ou un mouvement de l'utilisateur. Plus spécifiquement, un tel dispositif d'encodage 20 est particulièrement avantageux lorsqu'il est utilisé pour identifier un mouvement d'un utilisateur lorsqu'il (elle) porte une montre 11 ou un bracelet 12. Par exemple, un tel dispositif d'encodage 20 est particulièrement avantageux lorsqu'il est utilisé pour identifier une position ou un mouvement d'une semelle 13, 14 pour chaussure lorsqu'elle est insérée dans une chaussure ou intégrée à une chaussure.

Ne pesant que quelques grammes et étant de dimension réduite, ce dispositif d'encodage 20 peut se loger de façon peu encombrante dans n'importe quel accessoire par exemple une semelle intérieure et/ou extérieure. Ce faible volume limite l'impact sur le confort de l'utilisateur et présente l'avantage d'optimiser les coûts de production en rendant moins onéreux et plus simple l'intégration de cette technologie dans tout type d'accessoire porté par l'utilisateur ou directement dans l'entité mobile lors du processus industriel.

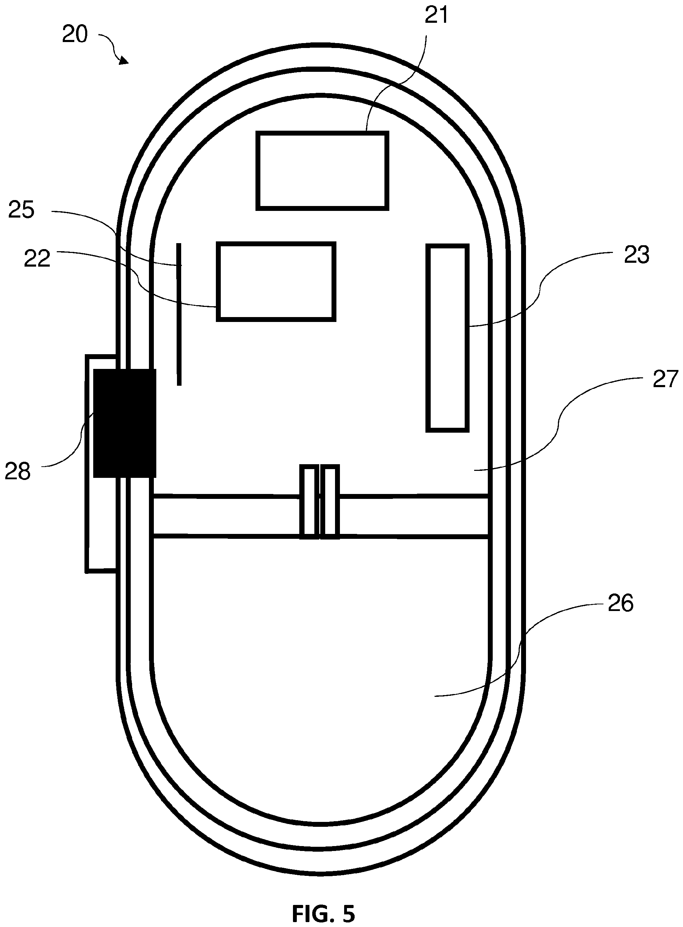

Un dispositif d'encodage 20 selon l'invention est détaillé dans la figure 5. Les différents composants du dispositif d'encodage 20 sont de préférence agencés sur une carte électronique 27 (ou circuit imprimé), mais l'invention peut prévoir divers types d'agencement, par exemple un seul module cumulant l'ensemble des fonctions décrites ici. De même, ces moyens peuvent être divisés en plusieurs cartes électroniques ou bien rassemblés sur une seule carte électronique. En outre, lorsque l'on prête une action à un dispositif, un moyen ou un module, celle-ci est en fait généralement effectuée par un microprocesseur du dispositif ou module commandé par des codes instructions enregistrés dans une mémoire. De même, si l'on prête une action à une application, celle-ci est en fait effectuée par un microprocesseur du dispositif dans une mémoire duquel les codes instructions correspondant à l'application sont enregistrés. Lorsqu'un dispositif ou module émet ou reçoit un message, ce message est émis ou reçu par une interface de communication.

Un tel dispositif d'encodage 20 selon l'invention peut comporter un capteur de mouvement 21 tel qu'une plateforme inertielle configurée pour générer des données sur le déplacement d'une entité mobile telle que la démarche d'un utilisateur. Au cours de l'utilisation du dispositif d'encodage 20 par un utilisateur (le dispositif d'encodage est avantageusement positionné sur un utilisateur), la plateforme inertielle acquière des signaux représentatifs d'un paramètre de mouvement (accélération et/ou vitesse, par exemple vitesse angulaire) de l'utilisateur, de préférence du pied de l'utilisateur, selon les axes X, Y, Z. En outre, ces données peuvent ensuite être traitées pour générer au moins un signal d'accélération. La plateforme inertielle est par exemple constituée d'au moins un accéléromètre et un gyroscope. De façon préférée, elle comporte plusieurs accéléromètres et gyroscopes. Le dispositif d'encodage 20 peut également comporter un ou plusieurs magnétomètres de façon à acquérir trois signaux bruts supplémentaires correspondant aux valeurs de champs magnétiques sur trois dimensions.

Chaque dispositif d'encodage 20 peut comporter par ailleurs d'autres capteurs, notamment un inclinomètre, un baromètre, un capteur de température et un altimètre pour bénéficier d'une précision accrue.

De façon préférée, un dispositif d'encodage 20 comporte un ou plusieurs processeurs 22 configurés pour exécuter un des procédés selon l'invention, de préférence le procédé d'encodage, et leurs différents modes de réalisation, préférés, avantageux ou non.

En particulier, les un ou plusieurs processeurs 22 sont configurés de façon à acquérir des données générées par le ou les capteurs de mouvement 21, lesdites données comportant des valeurs d'accélération et de vitesse angulaire.

Les un ou plusieurs processeurs 22 peuvent aussi être configurés de façon à calculer une pluralité de descripteurs à partir des données acquises sur une fenêtre temporelle 31, l'étape de calcul étant répétée pour plusieurs fenêtres temporelles 31 consécutives, les fenêtres temporelles 31 consécutives formant une séquence temporelle 32 ;

En outre, les un ou plusieurs processeurs 22 peuvent aussi être configurés de façon à transformer la pluralité de descripteurs calculés, ladite étape comportant un codage des descripteurs chacun sur au plus deux bits, lesdits bits associés à une fenêtre temporelle 31 étant représentatifs d'un phonème de mouvement.

Aussi, les un ou plusieurs processeurs 22 peuvent être configurés de façon à générer un tenseur de données comportant les bits obtenus pour chacune des fenêtres temporelles 31, de sorte que le tenseur de données comporte un encodage d'une combinaison de plusieurs phonèmes de mouvement qui est représentatif d'une posture ou d'un mouvement de l'utilisateur.

De façon préférée, les un ou plusieurs processeurs 22 seront configurées de façon à :

- acquérir des données générées par le ou les capteurs de mouvement 21,
- calculer une pluralité de descripteurs à partir des données acquises sur une fenêtre temporelle 31, l'étape de calcul étant répétée pour plusieurs fenêtres temporelles 31 consécutives ; et
- transformer la pluralité de descripteurs calculés, ladite étape comportant un codage des descripteurs chacun sur au plus deux bits, lesdits bits associés à une fenêtre temporelle 31 étant représentatifs d'un phonème de mouvement

En outre, les un ou plusieurs processeurs 22 seront configurées de façon à générer un tenseur de données comportant les bits obtenus pour chacune des fenêtres temporelles 31, de sorte que le tenseur de données comporte un encodage, de préférence d'une combinaison de plusieurs phonèmes de mouvement, qui est représentatif d'une position, d'une posture ou d'un mouvement de l'entité mobile (e.g. l'utilisateur).

En particulier, les un ou plusieurs processeurs 22 peuvent être configurés de façon à exécuter les actions suivantes :

- Charger des données codées sur au plus huit bits associées à la séquence temporelle 32, étant représentatives d'un mouvement d'une entité mobile sur ladite séquence temporelle 32, par exemple sous la forme d'un ou de plusieurs tenseurs de données ; et
- Traiter le ou les tenseurs de données par un modèle d'apprentissage automatique entrainé de manière identifier une position ou un mouvement de l'entité mobile.

Aussi, les un ou plusieurs processeurs 22 peuvent être configurés de façon à exécuter les actions suivantes : Charger des données codées sur au plus huit bits associées à la séquence temporelle 32, étant représentatives d'une position ou d'un mouvement de la chaussure sur ladite séquence temporelle 32, par exemple sous la forme d'un ou de plusieurs tenseurs de données ; et Traiter le ou les tenseurs de données par un modèle d'apprentissage automatique entrainé de manière identifier une posture ou un mouvement de l'utilisateur.

Alternativement, le ou les processeurs peuvent être configurés pour générer le tenseur de données puis l'envoyer à un terminal externe 30 pour que soit déterminée le déplacement de l'entité mobile (e.g. la démarche d'un utilisateur).

Ainsi, le dispositif d'encodage 20 selon l'invention comporte avantageusement un ou plusieurs moyens de communication 25. Les un ou plusieurs moyens de communication 25 sont aptes à recevoir et à transmettre les données sur au moins un réseau de communication. De préférence la communication est opérée par l'intermédiaire d'un protocole sans fils tel que wifi, 3G, 4G, 5G et/ou Bluetooth. De préférence le protocole de communication est un protocole BLE ou ANT+. Ces protocoles de communications permettent une consommation faible en énergie.

De préférence, le dispositif d'encodage 20 selon l'invention comporte un premier moyen de communication 25 configuré de sorte que le dispositif d'encodage 20 soit apte à transmettre au moins une partie du tenseur de données à un terminal externe 30. Ces données peuvent être transmises en temps réel ou en différé à un terminal externe 30. Le terminal externe 30 peut par exemple être un système distant tel qu'une tablette, un téléphone mobile (« smartphone » en terminologie anglo-saxonne), un ordinateur ou un serveur. Comme cela a été mentionné, le dispositif d'encodage 20 selon l'invention peut également mettre en oeuvre une étape de détermination du déplacement de l'entité mobile, telle que de la démarche d'un utilisateur. Ainsi, il peut être configuré pour envoyer des données de posture ou de mouvements identifiées pour un utilisateur. Ces données peuvent être transmises en temps réel ou en différé à un terminal externe 30.

Avantageusement, un dispositif d'encodage 20 selon l'invention comporte en outre un second moyen de communication configuré pour qu'un premier dispositif d'encodage 20 soit apte à communiquer avec un second dispositif d'encodage 20.

En particulier, les dispositifs d'encodage 20 peuvent être configurés pour communiquer entre eux et pour initier la génération de données sur le mouvement d'un utilisateur seulement après réception d'un message de l'autre dispositif d'encodage 20. Cela participe à la synchronisation des dispositifs d'encodage 20. Par exemple, les dispositifs d'encodage 20 peuvent être configurés pour associer et/ ou mettre en correspondance des horodatages des échantillons issus des plateformes inertielles.

Avantageusement, si un dispositif d'encodage 20 venait à se déconnecter ou perdre la synchronisation dans le temps par rapport à l'autre dispositif d'encodage 20, un procédé selon l'invention pourrait comprendre une étape de synchronisation des dispositifs d'encodage 20. Ainsi, un signal de recherche est envoyé par le dispositif d'encodage 20 connecté, le dispositif d'encodage 20 déconnecté reçoit le signal de recherche et se synchronise sur le dispositif d'encodage 20 connecté.

Le dispositif d'encodage 20 selon l'invention comporte également une mémoire de données 23, configurée pour mémoriser au moins une partie des données acquises et/ou des tenseurs de données. Le dispositif d'encodage 20 selon l'invention est tel qu'il permet de fonctionner avec une mémoire de données 23 de faible capacité. Il peut être configuré pour mémoriser les données brutes générées par la plateforme inertielle. Avantageusement, la mémoire de données 23 est configurée pour mémoriser au moins une partie des données transformées, mais ne pas mémoriser les données brutes générées. Ainsi, sa capacité n'est pas grevée par des données brutes générées. Les données transformées peuvent correspondre à des données prétraitées par le ou les processeurs ou à des tenseurs de données.

En outre, le dispositif d'encodage 20 selon l'invention peut comporter une source d'énergie 26. La source d'énergie est de préférence de type batterie, rechargeable ou non. De préférence la source d'énergie est une batterie rechargeable. En outre, elle peut être associée à un système de recharge par le mouvement ou par une énergie extérieure. Le système de recharge par une énergie extérieure peut notamment être un système de recharge par connexion filaire, un système de recharge par induction ou encore photovoltaïque.

La source d'énergie 26 est de préférence de type batterie, rechargeable ou non. De préférence la source d'énergie est une batterie rechargeable. La recharge peut être réalisée selon différentes technologies telles que :

- par chargeur, avec un connecteur affleurant au niveau de la montre, du bracelet ou de la semelle ;
- avec un dispositif de recharge mécanique intégré à la montre, du bracelet ou de la semelle, par exemple un dispositif piézoélectrique apte à fournir une énergie électrique à partir de la marche ;
- avec un dispositif sans contact, par exemple par induction ; et/ou
- avec un dispositif photovoltaïque.

En outre, le dispositif d'encodage selon l'invention peut comporter un port pour connexion filaire 28, de préférence protégé par une languette amovible. Ce port pour connexion filaire peut être par exemple un port USB ou firewire. Avantageusement, le port USB est également résistant à l'eau ou l'humidité. En outre, le port USB est avantageusement surmonté d'une poutrelle en polymère permettant de lui conférer une plus grande résistance en condition d'utilisation. Ce port pour connexion filaire 28 peut être utilisé comme évoqué ci-dessus pour recharger la batterie, mais également pour échanger des données et par exemple mettre à jour le micrologiciel de la carte électronique portant les différents composants du dispositif d'encodage.

De préférence, la languette amovible ou USB cover, permet de protéger le port USB de corps étrangers. Par exemple, la languette amovible permet de protéger le port USB de l'eau ou de la poussière. Une telle languette peut de préférence être réalisée en polymère de type élastomère ou polyuréthane.

En outre, selon un autre aspect, la présente invention porte sur un système de détermination 5 d'un déplacement d'une entité mobile, en particulier de la démarche d'un utilisateur.

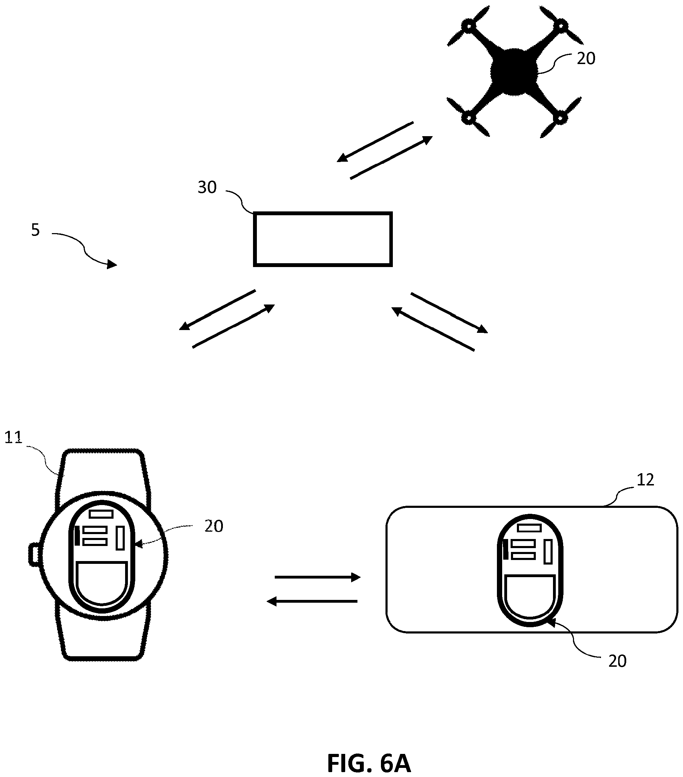

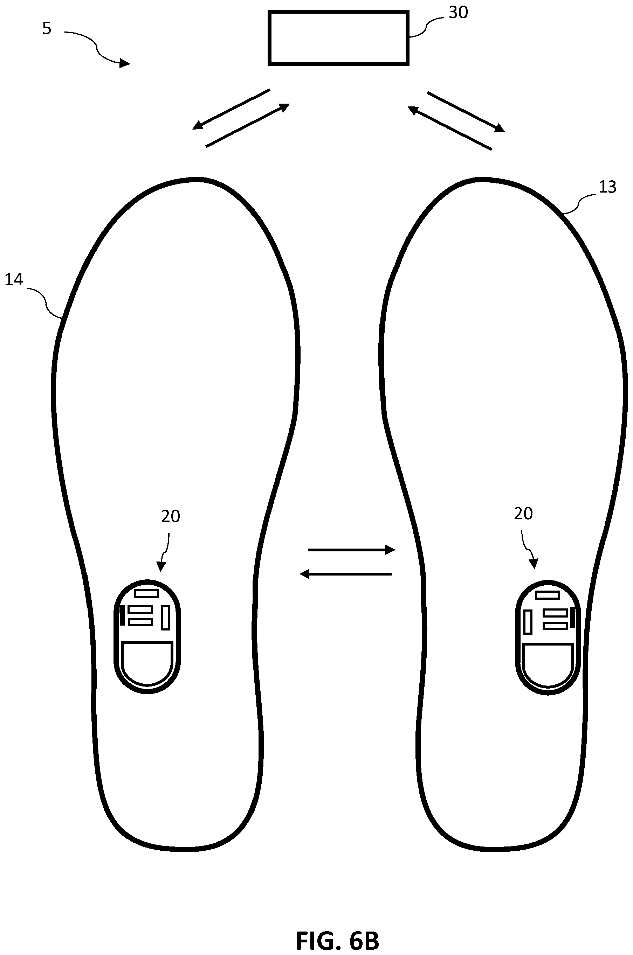

Un tel système de détermination 5, décrit en lien avec les figures 6A et 6B, comportera avantageusement un ou plusieurs dispositifs d'encodage 20 selon la présente invention. En particulier, le système de détermination 5 selon l'invention comporte au moins deux dispositifs d'encodage 20 selon la présente invention.

En particulier, chacun des dispositifs d'encodage 20 est conçu de manière à pouvoir communiquer indépendamment avec l'autre et/ou directement avec un terminal externe 30 afin de pouvoir échanger ses propres informations sur la posture/le mouvement/l'activité de l'utilisateur et en particulier de son pied.

Comme présenté sur la figure 6A, le système 5 selon l'invention peut comporter une montre 11 et un bracelet 12 ou bien deux bracelets 12, comportant chacun un dispositif d'encodage 20 selon l'invention. Classiquement, les montre 11 et bracelet 12 comportent chacun un dispositif d'encodage 20 selon l'invention. Comme présenté sur la figure 6B, le système 5 selon l'invention peut comporter une paire de semelles 13,14 comportant chacune un dispositif d'encodage 20 selon l'invention. Les semelles 13, 14 utilisables dans le cadre du système 5 selon l'invention peuvent par exemple correspondre à des semelles extérieures ou à des semelles intérieures, de chaussures. Ces semelles peuvent être amovibles ou être intégrées de manière permanente au semelage des chaussures. Classiquement, les semelles 13, 14 composants ladite paire de semelles, comportent chacune un dispositif d'encodage 20 selon l'invention. Comme présenté sur la figure 6B, le dispositif d'encodage 20 est de préférence positionné au niveau d'une portion médiane de la semelle.

Comme mentionné, le système 5 selon l'invention peut comporter un terminal externe 30 configuré pour recevoir des données provenant du ou des dispositif(s) d'encodage 20. Avantageusement, une application dédiée est installée sur ce terminal externe afin de traiter les informations transmises par le ou les dispositif(s) d'encodage 20 et permettre à l'utilisateur d'interagir avec l'invention. Il est alors par exemple possible d'accéder à ce serveur distant via une interface web. Toutes les communications avec le serveur distant peuvent être sécurisées par exemple par des protocoles HTTPS (pour « HyperText Transfer Protocol Secure » selon une terminologie anglo-saxonne) et cryptage AES (pour « Advanced Encryption Standard » selon une terminologie anglo-saxonne) 512. Ainsi, cela peut permettre, via un client, l'accès aux données par du personnel médical en charge du suivi de l'utilisateur.

Le terminal externe 30 est généralement une tablette, un téléphone mobile (« smartphone » en terminologie anglo-saxonne), une passerelle, un routeur, un ordinateur ou un serveur. Il peut être apte à transférer ces données à un serveur distant. Il est alors par exemple possible d'accéder à ce serveur distant via une interface web. Il pourra par exemple être configuré pour mettre en oeuvre des solutions d'inférence propres à des applications données à partir de données codées, ou de tenseurs de données codées ou en particulier de tenseurs de données d'ordre générés le dispositif d'encodage 20. Il pourra également être configuré pour générer des données sur le déplacement / la démarche à partir de données issues de l'étape de détermination 700 d'un déplacement de l'entité mobile et donc par exemple de la démarche de l'utilisateur.

Ainsi, l'utilisateur peut accéder à des données liées à son déplacement / sa démarche telle que des données relatives à ses activités physiques quotidiennes et relatives à plusieurs paramètres biomécaniques, comme la posture, la pronation/supination, la force d'impact, la longueur des pas, le temps de contact, la boiterie, l'équilibre et plusieurs autres paramètres relatifs à l'utilisateur et décrivant ses déplacements, sa marche, ses postures et ses mouvements et ainsi de suivre leur évolution.

## Revendications

1. Procédé (1) d'encodage de données générées par un ou plusieurs capteurs de mouvement (21) couplé(s) à une entité mobile, pour l'identification d'une position ou d'un mouvement de l'entité mobile sur une séquence temporelle (32),
ledit procédé (1), exécuté par un ou plusieurs processeurs (22), comportant :

   - Une étape d'acquisition (200) de données générées par le ou les capteurs de mouvement (21) couplé(s) à l'entité mobile, lesdites données comportant des valeurs variables sous forme de séries temporelles, de préférence lesdites valeurs variables comportent des valeurs d'accélération et de vitesse angulaire ;
   - Une étape de calcul (300), à partir des données acquises sur une fenêtre temporelle (31), d'une pluralité de descripteurs, l'étape de calcul (300) étant répétée pour plusieurs fenêtres temporelles (31) consécutives, des fenêtres temporelles (31) consécutives formant la séquence temporelle (32) ; et
   - Une étape de transformation (400) de la pluralité de descripteurs de chacune des fenêtres temporelles (31) consécutives formant la séquence temporelle (32), ladite étape de transformation (400) comportant une génération, pour chacun des descripteurs, d'une donnée codée sur au plus huit bits,

   lesdites données codées sur au plus huit bits associées à la séquence temporelle (32) étant représentatives d'une position ou d'un mouvement de l'entité mobile sur ladite séquence temporelle (32).

2. Procédé (1) d'encodage de données selon la revendication 1, **caractérisé en ce qu'**il comporte en outre une étape de génération (500) d'un tenseur de données (34) comportant les données codées sur au plus huit bits obtenus pour plusieurs des fenêtres temporelles (31), de sorte que le tenseur de données (34) comporte un encodage représentatif d'une position ou d'un mouvement de l'entité mobile.

3. Procédé (1) d'encodage de données selon l'une quelconque des revendications 1 ou 2, **caractérisé en ce que** les descripteurs sont calculés à partir de fonctions mathématiques appliquées aux données, prétraitées ou non, générées par le ou les capteurs de mouvement (21) couplé(s) à l'entité mobile, et lesdites fonctions mathématiques appliquées comportant par exemple un calcul de moyenne, d'asymétrie statistique, du coefficient d'aplatissement, de comparaisons de valeurs des extremums ou des quartiles, de positions des extremums dans une série temporelle, de comparaisons des valeurs de bord des sous-ensembles du mouvement et/ou un calcul d'une topologie de données.

4. Procédé (1) d'encodage de données selon l'une quelconque des revendications 1 à 3, **caractérisé en ce que** les fenêtres temporelles (31) de deux séquences temporelles (32) consécutives présentent des durées différentes.

5. Procédé (1) d'encodage de données selon l'une quelconque des revendications 1 à 4, **caractérisé en ce que**, lors de l'étape de transformation (400), au moins quatre descripteurs sont utilisés pour chacun générer une donnée codée sur au moins un bit, de façon plus préférée sur au moins deux bits.

6. Procédé (1) d'encodage de données selon l'une quelconque des revendications 1 à 5, **caractérisé en ce que**, lors de l'étape de transformation (400), entre quatre et vingt descripteurs sont utilisés pour chacun générer une donnée codée sur un nombre de bits sélectionné parmi : un, deux ou trois.

7. Procédé (1) d'encodage de données selon l'une quelconque des revendications 2 à 6, **caractérisé en ce qu'**il comporte une étape d'analyse des données codées sur au plus huit bits, de préférence du tenseur de données (34), en fonction d'une donnée d'activité de l'entité mobile, ladite étape d'analyse comprenant une comparaison entre les données codées sur au plus huit bits ou de préférence le tenseur de données (34) et une base de données comprenant des données de référence pour l'activité donnée.

8. Procédé (1) d'encodage de données selon l'une quelconque des revendications 1 à 7, **caractérisé en ce que**, lors de l'étape de transformation (400), une ou plusieurs données codées sur au plus huit bits, de préférence au plus trois bits, sont calculées à partir de valeurs de descripteurs calculés à partir de données acquises sur des fenêtres temporelles (31) consécutives.

9. Procédé (1) d'encodage de données selon l'une quelconque des revendications 1 à 8, **caractérisé en ce que** l'entité mobile est une chaussure, et l'étape d'acquisition est réalisée alors que les un ou plusieurs capteurs de mouvement ainsi que les un ou plusieurs processeurs sont intégrés dans un dispositif d'encodage, de préférence positionné dans une semelle.

10. Procédé de détermination (2) d'un déplacement d'une entité mobile, à partir de données susceptibles d'avoir été encodées selon un procédé d'encodage selon l'une quelconque des revendications 2 à 9, **caractérisé en ce que** ledit procédé de détermination (2), exécuté par un ou plusieurs processeurs (22), comporte :

   - Une étape de chargement (710) d'un ou de plusieurs tenseurs de données (34) comportant un encodage représentatif d'une position ou d'un mouvement de l'entité mobile, lesdits un ou de plusieurs tenseurs de données comportant une pluralité de descripteurs pour chacune des fenêtres temporelles (31) consécutives formant une séquence temporelle (32), chacun des descripteurs correspondant à une donnée codée sur au plus huit bits ; et
   - Une étape de traitement (720) du ou des tenseurs de données (34) par un modèle d'apprentissage automatique entrainé de manière à identifier une position ou un mouvement de l'entité mobile.

11. Procédé de détermination (2) d'un déplacement d'une entité mobile selon la revendication 10, **caractérisé en ce que** l'étape de traitement (720) du ou des tenseurs de données par un modèle d'apprentissage automatique comporte l'identification de classes d'événements à partir du ou des tenseurs de données (34), de préférence une classe de mouvement par séquence temporelle (32).

12. Procédé de détermination (2) d'un déplacement d'une entité mobile selon l'une quelconque des revendications 10 ou 11, **caractérisé en ce qu'**il comporte une étape de sélection (730), de préférence par le modèle d'apprentissage automatique entraîné, d'une position et/ou d'un mouvement à partir d'une comparaison des positions et/ou des mouvements identifiés à partir de fenêtres temporelles (31) contiguës ou de séquences temporelles (32) contiguës.

13. Procédé de détermination (2) d'un déplacement d'une entité mobile selon l'une quelconque des revendications 10 à 12, **caractérisé en ce qu'**il comporte une étape de comparaison (740) des positions et/ou des mouvements identifiés pour une fenêtre temporelle (31) ou une séquence temporelle (32) donnée à partir de données de capteurs différents de façon à identifier une position et/ou un mouvement de l'entité mobile.

14. Procédé de détermination (2) d'un déplacement d'une entité mobile selon l'une quelconque des revendications 10 à 13, **caractérisé en ce que** l'entité mobile est un utilisateur d'une chaussure, l'encodage représentatif d'une position ou d'un mouvement de l'entité mobile est un encodage représentatif d'une position ou d'un mouvement de la chaussure de l'utilisateur ; et l'étape de traitement (720) du ou des tenseurs de données permet d'identifier une posture ou un mouvement de l'utilisateur.

15. Dispositif d'encodage (20) de données générées par un ou plusieurs capteurs de mouvement (21) couplé(s) à une entité mobile, pour l'identification d'une position ou d'un mouvement de l'entité mobile sur une séquence temporelle (32), ledit dispositif d'encodage (20) comportant un ou plusieurs processeurs (22), lesdits un ou plusieurs processeurs (22) étant configurés pour :

   - Acquérir les données générées par le ou les capteurs de mouvement (21) couplé(s) à l'entité mobile, lesdites données comportant des valeurs variables sous forme de séries temporelles, de préférence lesdites valeurs variables comportent des valeurs d'accélération et de vitesse angulaire ;

- Calculer, à partir des données acquises sur une fenêtre temporelle (31), une pluralité de descripteurs, le calcul étant répété pour plusieurs fenêtres temporelles (31) consécutives, les fenêtres temporelles (31) consécutives formant une séquence temporelle (32) ;

- Transformer la pluralité de descripteurs de chacune des fenêtres temporelles (31) consécutives formant la séquence temporelle (32), ladite transformation comportant une génération pour chacun des descripteurs d'une donnée codée sur au plus huit bits, lesdites données codées sur au plus huit bits associées à la séquence temporelle (32) étant représentatives d'une position ou d'un mouvement de l'entité mobile sur ladite séquence temporelle (32).

16. Dispositif d'encodage (20) de données générées par un ou plusieurs capteurs de mouvement (21) selon la revendication précédente **caractérisé en ce que** lesdits un ou plusieurs processeurs (22) sont configurés pour générer un tenseur de données (34) comportant les données codées sur au plus huit bits obtenues pour plusieurs des fenêtres temporelles (31), de sorte que le tenseur de données (34) comporte un encodage représentatif d'une position ou d'un mouvement de l'entité mobile.

17. Dispositif d'encodage (20) de données générées par un ou plusieurs capteurs de mouvement (21) selon l'une des revendications 15 ou 16, **caractérisé en ce qu'**il comporte lesdits un ou plusieurs capteurs de mouvement (21).

18. Dispositif d'encodage (20) de données générées par un ou plusieurs capteurs de mouvement (21) selon l'une quelconque des revendications 15 à 17, **caractérisé en ce qu'**il est intégré dans une semelle (13,14) amovible ou non.

19. Dispositif d'encodage (20) de données générées par un ou plusieurs capteurs de mouvement (21) selon l'une quelconque des revendications 15 à 18, **caractérisé en ce que** lesdits un ou plusieurs capteurs de mouvement (21) comportent au moins un accéléromètre et au moins un gyroscope.

20. Système (5) de détermination d'un déplacement d'une entité mobile, ledit système de détermination comportant au moins un dispositif d'encodage (20) selon l'une quelconque des revendications 15 à 19 et un terminal externe (30) configuré pour recevoir le tenseur de données (34) généré puis pour déterminer le déplacement d'une entité mobile à partir du tenseur de données (34) généré.

**Patentansprüche**

1. Verfahren (1) zur Kodierung von Daten, die von einem oder mehreren Bewegungssensor(en) (21) erzeugt werden, die mit einer mobilen Einheit gekoppelt sind, zur Identifizierung einer Position oder Bewegung der mobilen Einheit über eine zeitliche Sequenz (32), wobei das Verfahren (1), ausgeführt von einem oder mehreren Prozessoren (22), umfasst:

- Einen Schritt des Erwerbs (200) von Daten, die von dem oder den Bewegungssensor(en) (21) erzeugt werden, die mit der mobilen Einheit gekoppelt sind, wobei diese Daten variable Werte in Form von Zeitreihen enthalten, vorzugsweise umfassen diese variablen Werte Beschleunigungs- und Winkelgeschwindigkeitswerte;

- Einen Berechnungsschritt (300), bei dem aus den über ein Zeitfenster (31) erfassten Daten eine Vielzahl von Deskriptoren berechnet wird, wobei der Berechnungsschritt (300) für mehrere aufeinanderfolgende Zeitfenster (31) wiederholt wird, die zusammen die zeitliche Sequenz (32) bilden; und

- Einen Transformationsschritt (400) der Vielzahl von Deskriptoren jedes der aufeinanderfolgenden Zeitfenster (31), die die zeitliche Sequenz (32) bilden,

wobei dieser Transformationsschritt (400) eine Generierung von auf höchstens acht Bits codierten Daten für jeden der Deskriptoren umfasst,

wobei die mit der zeitlichen Sequenz (32) assoziierten auf höchstens acht Bits codierten Daten repräsentativ für eine Position oder Bewegung der mobilen Einheit über die genannte zeitliche Sequenz (32) sind.

2. Verfahren (1) zur Kodierung von Daten nach Anspruch 1, **dadurch gekennzeichnet, dass** es ferner einen Schritt der Generierung (500) eines Datentensors (34) umfasst, der die für mehrere der Zeitfenster (31) erhaltenen auf höchstens acht Bits codierten Daten enthält, sodass der Datentensor (34) eine Kodierung enthält, die repräsentativ für eine Position oder Bewegung der mobilen Einheit ist.

3. Verfahren (1) zur Kodierung von Daten nach einem der Ansprüche 1 oder 2, **dadurch gekennzeichnet, dass** die Deskriptoren aus mathematischen Funktionen berechnet werden, die auf die vorverarbeiteten oder nicht vorverarbeiteten Daten angewendet werden, die von dem oder den Bewegungssensor(en) (21) erzeugt werden, die mit der mobilen Einheit gekoppelt sind, wobei die genannten angewendeten mathematischen Funktionen beispielsweise eine Berechnung des Mittelwerts, der statistischen Schiefe, des Kurtosis-Koeffizienten, Vergleiche von Extrem- oder Quartilswerten, Positionen der Extrema in einer Zeitreihe, Vergleiche der Randwerte der Bewegungsteilbereiche und/oder eine Berechnung einer Datentopologie umfassen.

4. Verfahren (1) zur Kodierung von Daten nach einem der Ansprüche 1 bis 3, **dadurch gekennzeichnet, dass** die Zeitfenster (31) von zwei aufeinanderfolgenden zeitlichen Sequenzen (32) unterschiedliche Dauern aufweisen.

5. Verfahren (1) zur Kodierung von Daten nach einem der Ansprüche 1 bis 4, **dadurch gekennzeichnet, dass** während des Transformationsschritts (400) mindestens vier Deskriptoren verwendet werden, um jeweils Daten zu erzeugen, die auf mindestens einem Bit codiert sind, vorzugsweise auf mindestens zwei Bits.

6. Verfahren (1) zur Kodierung von Daten nach einem der Ansprüche 1 bis 5, **dadurch gekennzeichnet, dass** während des Transformationsschritts (400) zwischen vier und zwanzig Deskriptoren verwendet werden, um jeweils eine Daten zu erzeugen, die auf einer Anzahl von Bits codiert ist, ausgewählt aus: eins, zwei oder drei.

7. Verfahren (1) zur Kodierung von Daten nach einem der Ansprüche 2 bis 6, **dadurch gekennzeichnet, dass** es einen Schritt der Analyse der auf höchstens acht Bits codierten Daten, vorzugsweise des Datentensors (34), in Abhängigkeit von einer Aktivitätsdaten der mobilen Einheit umfasst, wobei dieser Analyseschritt einen Vergleich zwischen den auf höchstens acht Bits codierten Daten oder vorzugsweise dem Datentensor (34) und einer Datenbank mit Referenzdaten für die gegebene Aktivität umfasst.

8. Verfahren (1) zur Kodierung von Daten nach einem der Ansprüche 1 bis 7, **dadurch gekennzeichnet, dass** während des Transformationsschritts (400) eine oder mehrere auf höchstens acht Bits codierte Daten, vorzugsweise auf höchstens drei Bits, aus Werten von Deskriptoren berechnet werden, die aus Daten berechnet wurden, die über aufeinanderfolgende Zeitfenster (31) erfasst wurden.

9. Verfahren (1) zur Kodierung von Daten nach einem der Ansprüche 1 bis 8, **dadurch gekennzeichnet, dass** die mobile Einheit ein Schuh ist und der Erwerbsschritt durchgeführt wird, während der oder die Bewegungssensor(en) sowie der oder die Prozessor(en) in eine Kodierungsvorrichtung integriert sind, vorzugsweise in einer Sohle positioniert.

10. Verfahren (2) zur Bestimmung einer Bewegung einer mobilen Einheit aus Daten, die möglicherweise nach einem Kodierungsverfahren nach einem der Ansprüche 2 bis 9 kodiert wurden, **dadurch gekennzeichnet, dass** dieses Bestimmungsverfahren (2), ausgeführt von einem oder mehreren Prozessoren (22), umfasst:

   - Einen Schritt des Ladens (710) eines oder mehrerer Datentensoren (34), die eine Kodierung enthalten, die repräsentativ für eine Position oder Bewegung der mobilen Einheit ist, wobei diese ein oder mehreren Datentensoren eine Vielzahl von Deskriptoren für jedes der aufeinanderfolgenden Zeitfenster (31) umfassen, die eine zeitliche Sequenz (32) bilden, wobei jeder der Deskriptoren einer auf höchstens acht Bits codierten Daten entspricht; und
   - Einen Verarbeitungsschritt (720) des oder der Datentensoren (34) durch ein trainiertes maschinelles Lernmodell, um eine Position oder Bewegung der mobilen Einheit zu identifizieren.

11. Verfahren (2) zur Bestimmung einer Bewegung einer mobilen Einheit nach Anspruch 10, **dadurch gekennzeichnet, dass** der Verarbeitungsschritt (720) des oder der Datentensoren durch ein maschinelles Lernmodell die Identifizierung von Ereignisklassen aus dem oder den Datentensoren (34) umfasst, vorzugsweise eine Bewegungsklasse pro zeitlicher Sequenz (32).

12. Verfahren (2) zur Bestimmung einer Bewegung einer mobilen Einheit nach einem der Ansprüche 10 oder 11, **dadurch gekennzeichnet, dass** es einen Schritt der Auswahl (730), vorzugsweise durch das trainierte maschinelle Lernmodell, einer Position und/oder Bewegung aus einem Vergleich der identifizierten Positionen und/oder Bewegungen aus benachbarten Zeitfenstern (31) oder benachbarten zeitlichen Sequenzen (32) umfasst.

13. Verfahren (2) zur Bestimmung einer Bewegung einer mobilen Einheit nach einem der Ansprüche 10 bis 12, **dadurch**

**gekennzeichnet, dass** es einen Schritt des Vergleichs (740) der für ein gegebenes Zeitfenster (31) oder eine gegebene zeitliche Sequenz (32) identifizierten Positionen und/oder Bewegungen aus Daten unterschiedlicher Sensoren umfasst, um eine Position und/oder Bewegung der mobilen Einheit zu identifizieren.

14. Verfahren (2) zur Bestimmung einer Bewegung einer mobilen Einheit nach einem der Ansprüche 10 bis 13, **dadurch gekennzeichnet, dass** die mobile Einheit ein Benutzer eines Schuhs ist, die Kodierung, die repräsentativ für eine Position oder Bewegung der mobilen Einheit ist, eine Kodierung ist, die repräsentativ für eine Position oder Bewegung des Schuhs des Benutzers ist; und dass der Verarbeitungsschritt (720) des oder der Datentensoren es ermöglicht, eine Haltung oder Bewegung des Benutzers zu identifizieren.

15. Kodierungsvorrichtung (20) für von einem oder mehreren Bewegungssensor(en) (21) erzeugte Daten, die mit einer mobilen Einheit gekoppelt sind, zur Identifizierung einer Position oder Bewegung der mobilen Einheit über eine zeitliche Sequenz (32), wobei diese Kodierungsvorrichtung (20) einen oder mehrere Prozessoren (22) umfasst, die so konfiguriert sind, dass sie:

- Die von dem oder den Bewegungssensor(en) (21) erzeugten Daten, die mit der mobilen Einheit gekoppelt sind, erfassen, wobei diese Daten variable Werte in Form von Zeitreihen enthalten, vorzugsweise umfassen diese variablen Werte Beschleunigungs- und Winkelgeschwindigkeitswerte;
- Aus den über ein Zeitfenster (31) erfassten Daten eine Vielzahl von Deskriptoren berechnen, wobei die Berechnung für mehrere aufeinanderfolgende Zeitfenster (31) wiederholt wird, die zusammen die zeitliche Sequenz (32) bilden;
- Die Vielzahl von Deskriptoren jedes der aufeinanderfolgenden Zeitfenster (31), die die zeitliche Sequenz (32) bilden, transformieren, wobei diese Transformation die Generierung von auf höchstens acht Bits codierten Daten für jeden der Deskriptoren umfasst, wobei die mit der zeitlichen Sequenz (32) assoziierten auf höchstens acht Bits codierten Daten repräsentativ für eine Position oder Bewegung der mobilen Einheit über die genannte zeitliche Sequenz (32) sind.

16. Kodierungsvorrichtung (20) für von einem oder mehreren Bewegungssensor(en) (21) erzeugte Daten nach dem vorhergehenden Anspruch, **dadurch gekennzeichnet, dass** die genannten ein oder mehreren Prozessoren (22) so konfiguriert sind, dass sie einen Datentensor (34) generieren, der die für mehrere der Zeitfenster (31) erhaltenen auf höchstens acht Bits codierten Daten enthält, sodass der Datentensor (34) eine Kodierung enthält, die repräsentativ für eine Position oder Bewegung der mobilen Einheit ist.

17. Kodierungsvorrichtung (20) für von einem oder mehreren Bewegungssensor(en) (21) erzeugte Daten nach einem der Ansprüche 15 oder 16, **dadurch gekennzeichnet, dass** sie die genannten ein oder mehreren Bewegungssensor(en) (21) umfasst.

18. Kodierungsvorrichtung (20) für von einem oder mehreren Bewegungssensor(en) (21) erzeugte Daten nach einem der Ansprüche 15 bis 17, **dadurch gekennzeichnet, dass** sie in eine abnehmbare oder nicht abnehmbare Sohle (13,14) integriert ist.

19. Kodierungsvorrichtung (20) für von einem oder mehreren Bewegungssensor(en) (21) erzeugte Daten nach einem der Ansprüche 15 bis 18, **dadurch gekennzeichnet, dass** die genannten ein oder mehreren Bewegungssensor(en) (21) mindestens einen Beschleunigungsmesser und mindestens ein Gyroskop umfassen.

20. System (5) zur Bestimmung einer Bewegung einer mobilen Einheit, wobei dieses Bestimmungssystem mindestens eine Kodierungsvorrichtung (20) nach einem der Ansprüche 15 bis 19 und ein externes Terminal (30) umfasst, das so konfiguriert ist, dass es den generierten Datentensor (34) empfängt und dann die Bewegung einer mobilen Einheit aus dem generierten Datentensor (34) bestimmt.

**Claims**

1. A method (1) for encoding data generated by one or several movement sensors (21) coupled to a moving entity, for the identification of a position or movement of the moving entity on a time sequence (32),

   said method (1), executed by one or several processors (22), including:

- a step (200) of acquiring data generated by the movement sensor(s) (21) coupled to the moving entity, said data including variable values in the form of time series, preferably said variable values include acceleration and angular speed values;
- a step (300) of calculating, from the data acquired over a time window (31), a plurality of descriptors, the calculation step (300) being repeated for several consecutive time windows (31), the consecutive time windows (31) forming the time sequence (32); and
- a step (400) of transforming the plurality of descriptors of each of the consecutive time windows (31) forming the time sequence (32),

said transformation step (400) including a generation, for each of the descriptors, of a data coded on at most eight bits,
said data coded on at most eight bits associated with the time sequence (32) being representative of a position or movement of the moving entity on said time sequence (32).

2. The data encoding method (1) according to claim 1, **characterized in that** it further includes a step (500) of generating a data tensor (34) including the data coded on at most eight bits obtained for several of the time windows (31), so that the data tensor (34) includes an encoding representative of a position or movement of the moving entity.

3. The data encoding method (1) according to any one of claims 1 or 2, **characterized in that** the descriptors are calculated from mathematical functions applied to the preprocessed or non-preprocessed data generated by the movement sensor(s) (21) coupled to the moving entity, and said applied mathematical functions including for example a calculation of average, of statistical asymmetry, of the Kurtosis coefficient, of comparisons of values of the extrema or the quartiles, of positions of the extrema in a time series, of comparisons of the boundary values of the movement subsets and/or of a calculation of a data topology.

4. The data encoding method (1) according to any one of claims 1 to 3, **characterized in that** the time windows (31) of two consecutive time sequences (32) have different durations.

5. The data encoding method (1) according to any one of claims 1 to 4, **characterized in that**, during the transformation step (400), at least four descriptors are used to each generate a data coded on at least one bit, more preferably on at least two bits.

6. The data encoding method (1) according to any one of claims 1 to 5, **characterized in that**, during the transformation step (400), between four and twenty descriptors are used to each generate a data coded on a number of bits selected from: one, two or three.

7. The data encoding method (1) according to any one of claims 2 to 6, **characterized in that** it includes a step of analyzing the data coded on at most eight bits, preferably of the data tensor (34), as a function of an activity data of the moving entity, said analysis step comprising a comparison between the data coded on at most eight bits or preferably the data tensor (34) and a database comprising reference data for the given activity.

8. The data encoding method (1) according to any one of claims 1 to 7, **characterized in that**, during the transformation step (400), one or several data coded on at most eight bits, preferably at most three bits, are calculated from values of descriptors calculated from data acquired over consecutive time windows (31).

9. The data encoding method (1) according to any one of claims 1 to 8, **characterized in that** the moving entity is a shoe, and the acquisition step is carried out while the one or several movement sensors as well as the one or several processors are integrated in an encoding device, preferably positioned in a sole.

10. A method (2) for determining a displacement of a moving entity, from data likely to have been encoded according to an encoding method according to any one of claims 2 to 9, **characterized in that** said determination method (2), executed by one or several processors (22), includes:

- a step of loading (710) one or several data tensors (34) including an encoding representative of a position or movement of the moving entity, said one or several data tensors including a plurality of descriptors for each of the consecutive time windows (31) forming a time sequence (32), each of the descriptors corresponding to a data coded on at most eight bits; and
- a step (720) of processing the data tensor(s) (34) by a machine learning model trained so as to identify a position or movement of the moving entity.

11. The method (2) for determining a displacement of a moving entity according to claim 10, **characterized in that** the step (720) of processing the data tensor(s) by a machine learning model includes the identification of classes of events from the data tensor(s) (34), preferably one class of movement per time sequence (32) .

12. The method (2) for determining a displacement of a moving entity according to any one of claims 10 or 11, **characterized in that** it includes a step (730) of selecting, preferably by the trained machine learning model, a position and/or movement from a comparison of the positions and/or movements identified from contiguous time windows (31) or contiguous time sequences (32).

13. The method (2) for determining a displacement of a moving entity according to any one of claims 10 to 12, **characterized in that** it includes a step (740) of comparing the positions and/or movements identified for a given time window (31) or time sequence (32) from data from different sensors so as to identify a position and/or movement of the moving entity.

14. The method (2) for determining a displacement of a moving entity according to any one of claims 10 to 13, **characterized in that** the moving entity is a user of a shoe, the encoding representative of a position or movement of the moving entity is an encoding representative of a position or movement of the shoe of the user; and the step (720) of processing the data tensor(s) makes it possible to identify a posture or movement of the user.

15. A device (20) for encoding data generated by one or several movement sensors (21) coupled to a moving entity, for the identification of a position or movement of the moving entity on a time sequence (32), said encoding device (20) including one or several processors (22), said one or several processors (22) being configured to:

   - acquire the data generated by the movement sensor(s) (21) coupled to the moving entity, said data including variable values in the form of time series, preferably said variable values include acceleration and angular speed values;
   - calculate, from the data acquired over a time window (31), a plurality of descriptors, the calculation being repeated for several consecutive time windows (31), the consecutive time windows (31) forming a time sequence (32);
   - transform the plurality of descriptors of each of the consecutive time windows (31) forming the time sequence (32), said transformation including a generation for each of the descriptors of a data coded on at most eight bits, said data coded on at most eight bits associated with the time sequence (32) being representative of a position or movement of the moving entity on said time sequence (32).

16. The device (20) for encoding data generated by one or several movement sensors (21) according to the preceding claim **characterized in that** said one or several processors (22) are configured to generate a data tensor (34) including the data coded on at most eight bits obtained for several of the time windows (31), such that the data tensor (34) includes an encoding representative of a position or movement of the moving entity.

17. The device (20) for encoding data generated by one or several movement sensors (21) according to any of claims 15 or 16, **characterized in that** it includes said one or several movement sensors (21).

18. The device (20) for encoding data generated by one or several movement sensors (21) according to any one of claims 15 to 17, **characterized in that** it is integrated in a removable or non-removable sole (13, 14).

19. The device (20) for encoding data generated by one or several movement sensors (21) according to any one of claims 15 to 18, **characterized in that** said one or several movement sensors (21) include at least an accelerometer and at least one gyroscope.

20. A system (5) for determining a displacement of a moving entity, said determination system including at least one encoding device (20) according to any one of claims 15 to 19 and an external terminal (30) configured to receive the generated data tensor (34) and then to determine the displacement of a moving entity from the generated data tensor (34).

200 — Acquisition des données générées par le ou les capteurs de mouvement

300 — Calcul d'une pluralité de descripteurs à partir des données acquises sur une fenêtre temporelle

400 — Transformation de la pluralité de descripteurs avec codage des descripteurs chacun sur au plus huit bits

500 — Génération d'un tenseur de données comportant les bits obtenus pour chacune des fenêtres temporelles

700 — Détermination d'un déplacement de l'entité mobile

800 — Identification d'une activité associée à un ou plusieurs mouvements et/ou positions identifiés

**FIG. 1**

100 — Apprentissage

200 — Acquisition des données générées par le ou les capteurs de mouvement

210 — Calibration

220 — Prétraitement

230 — Extraction d'une séquence temporelle correspondant à une unité de mouvement

300 — Calcul d'une pluralité de descripteurs à partir des données acquises sur une fenêtre temporelle

400 — Transformation de la pluralité de descripteurs avec codage des descripteurs chacun sur au plus deux bits

500 — Génération d'un vecteur de données comportant les bits obtenus pour chacune des fenêtres temporelles

600 — Transmission du tenseur de données

700 — Détermination d'un déplacement de l'entité mobile

900 — Mémorisation des données

**FIG. 2**

**FIG. 3A**

FIG. 3B

FIG. 3C

34

710 — Chargement d'un tenseur de données

720 — Traitement du tenseur de données par un modèle d'apprentissage entrainé

730 — Sélection d'une position ou d'un mouvement à partir des positions ou des mouvements identifiés pour des séquences temporelles contiguës

740 — Comparaison des positions et/ou des mouvements identifiés à partir de données de capteurs différents.

750 — Transmission de données à un terminal externe

**FIG. 4**

**FIG. 5**

**FIG. 6A**

**FIG. 6B**

## RÉFÉRENCES CITÉES DANS LA DESCRIPTION

**Documents brevets cités dans la description**

- US 2007061105 A
- US 10978093 B
- WO 2019193301 A
- WO 2019077266 A
- WO 2020217037 A

**Littérature non-brevet citée dans la description**

- **SALMA SAIDANI et al.** A survey on smart shoe insole systems. IEEE, 2018
- **NIMSIRI ABHAYASINGHE et al.** Conference: Indoor Positioning and Indoor Navigation. *Bluetooth Embedded Inertial Measurement Unit for Real-Time Data Collection for Gait Analysis,* Octobre 2013
- **H. EDELSBRUNNER ; J. HARER.** Computational Topology. An Introduction. *American Mathematical Society,* 2009
- **H. EDELSBRUNNER ; D. LETSCHER ; A. ZO-MORODIAN.** Topological persistence and simplification. *Disc. Compu. Geom,* 2002
- **J. TIERNY ; A. GYULASSY ; E. SIMON ; V. PAS-CUCCI.** Loop surgery for volu-metric meshes: Reeb graphs reduced to contour trees. *IEEE Transactionson Visualization and Computer Graphics (Proc. of IEEE VIS),* 2009
- **A. GYULASSY ; P. T. BREMER ; B. HAMANN ; V. PASCUCCI.** A practical approach to morse-smale complex computation: Scalability and generality. *IEEE Transactions on Visualization and Computer Graphics (Proc. Of IEEE VIS),* 2008